(19) Europäisches Patentamt
European Patent Office
Office européen des brevets

(11) **EP 3 181 057 A1**

(12) **EUROPEAN PATENT APPLICATION**
published in accordance with Art. 153(4) EPC

(43) Date of publication:
**21.06.2017 Bulletin 2017/25**

(51) Int Cl.:
***A61B 8/13*** (2006.01)

(21) Application number: **15832033.3**

(22) Date of filing: **03.07.2015**

(86) International application number:
**PCT/JP2015/069286**

(87) International publication number:
**WO 2016/024449 (18.02.2016 Gazette 2016/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **12.08.2014 JP 2014164026**
**28.08.2014 JP 2014173837**

(71) Applicant: **PreXion Corporation**
**Chiyoda-ku**
**Tokyo 101-0041 (JP)**

(72) Inventors:
• **NAKATSUKA, Hitoshi**
**Tokyo 101-0041 (JP)**
• **KITAGAWA, Kazuo**
**Tokyo 101-0041 (JP)**
• **HANAOKA, Takamitsu**
**Tokyo 101-0041 (JP)**

(74) Representative: **Grünecker Patent- und Rechtsanwälte**
**PartG mbB**
**Leopoldstraße 4**
**80802 München (DE)**

(54) **PHOTO-ACOUSTIC IMAGING DEVICE**

(57)    This photo-acoustic imaging device (100) is provided with: light emitting diodes (16); an acoustic wave detecting unit (14); a device main body (2) in which a light source driving unit (22) including a power supply unit (22a) and a signal generating unit (22b) is provided; and a coaxial cable (3). For the coaxial cable (3), an external conductor (3c) of the coaxial cable (3) is connected to the power supply unit (22a) and an internal conductor (3a) of the coaxial cable (3) is connected to the signal generating unit (22b).

Fig 4

EP 3 181 057 A1

**Description**

Technical Field

The present invention relates to a photoacoustic imaging apparatus, particularly, to a photoacoustic imaging apparatus including a probe with a detecting portion.

Background Art

A photoacoustic imaging apparatus including a probe with a detecting portion has conventionally been known. Such a photoacoustic imaging apparatus is disclosed in Japanese Patent Laying-Open No. 2013-188330, for example.
Japanese Patent Laying-Open No. 2013-188330 discloses a test object information acquiring apparatus including a probe with an ultrasonic probe. This test object information acquiring apparatus includes a light source, the probe with an emitting portion and the ultrasonic probe, and a processor. The emitting portion is configured to guide pulsed light from the light source arranged separately from the emitting portion to a test object. The ultrasonic probe is configured to acquire an acoustic wave generated when the pulsed light is emitted from the emission part to the test object. The processor is configured to form an image of the acoustic wave acquired by the ultrasonic probe.
An image forming apparatus including a connection cable has conventionally been known. Such a photoacoustic imaging apparatus is disclosed in Japanese Patent Laying-Open No. 2008-44148, for example.
Japanese Patent Laying-Open No. 2008-44148 discloses an image forming apparatus including a connection cable. This image forming apparatus includes a print control portion, the connection cable, and an LED head. The print control portion and the LED head are connected through the connection cable. The LED head includes an input portion resistor and a termination resistor and is configured in such a manner that the impedance of the LED head and the impedance (characteristic impedance) of the connection cable are matched. This achieves a configuration of reducing the likelihood of the occurrence of a reflected wave of a control signal from the print control portion due to failing to match the impedance of the LED head and the impedance of the connection cable.
If the light source is provided to the probe in the test object information acquiring apparatus of Japanese Patent Laying-Open No. 2013-188330 with the intention of reducing loss of the quantity of light to be emitted (guided) from the light source to the test object, power (pulsed power) should be supplied to the light source through the connection cable from an apparatus body (light source drive portion) of the test object information acquiring apparatus. In this case, the occurrence of a reflected wave of the pulsed power may be avoided by a configuration of providing the input portion resistor and the termination resistor of Japanese Patent Laying-Open No. 2008-44148 to the light source.

Citation List

Patent Literatures

Patent Literature 1: Japanese Patent Laying-Open No. 2013-188330
Patent Literature 2: Japanese Patent Laying-Open No. 2008-44148

Summary of Invention

Technical Problem

However, in the test object information acquiring apparatus of Japanese Patent Laying-Open No. 2013-188330 where the input portion resistor and the termination resistor of Japanese Patent Laying-Open No. 2008-44148 are provided to the light source, provision of the input portion resistor and the termination resistor is considered reduce the responsivity of a current flowing in the light source (light-emitting element) relative to the pulsed power (pulse emission signal). Further, in the aforementioned test object information acquiring apparatus, the pulse emission signal is transmitted through the connection cable. Hence, the waveform of the pulse emission signal is considered to be disturbed due to entry of an electromagnetic wave, etc. (noise) from the outside of the connection cable (from a different device, for example) into the inside of the connection cable. It is also likely that an electromagnetic wave will be emitted from the inside to the outside of the connection cable and the emitted electromagnetic wave will influence the light source. As a result, reduction in the responsivity of a current flowing in the light source (light-emitting element) and the disturbed waveform of the pulse emission signal are considered to inhibit a sufficient flow of a current in the light-emitting element, thereby causing shortage of the quantity of light emitted from the light-emitting element. Thus, the test object information acquiring apparatus of Japanese Patent Laying-Open No. 2013-188330, including the input portion resistor and the termination resistor of Japanese Patent Laying-Open No. 2008-44148 in the light source, is considered to have a problem of shortage of the quantity of light emitted from the light-emitting element due to reduction in the responsivity of a current flowing in the light-emitting element and an electromagnetic wave, etc. (noise) coming from outside. In this description, the responsivity of a current flowing in the light-emitting element is determined by the sum of time from application of the pulse emission signal (voltage) to the light-emitting element to a moment when the value of the current flowing in the light-emitting element becomes a substantially peak value, and time from stop of the pulse emission signal to a moment when the value of the current flowing in the light-emitting element becomes substantially zero.

The present invention has been made to solve the above-described problem. It is one object of the present invention to provide a photoacoustic imaging apparatus capable of suppressing shortage of the quantity of light emitted from a light-emitting element by reducing the likelihood of entry of an electromagnetic wave, etc. (noise) from outside while suppressing reduction in the responsivity of a current flowing in the light-emitting element, and by suppressing inside-to-outside emission of an electromagnetic wave.

Solution to Problem

To attain the aforementioned object, a photoacoustic imaging apparatus according to one aspect of the present invention includes: a light-emitting element capable of emitting light to a test object; a detecting portion that detects an acoustic wave generated by absorption of light by a detection target in the test object, the light being light emitted from the light-emitting element to the test object; an apparatus body with a light source drive portion that includes a power supply portion and a signal generating portion, the power supply portion supplying power to the light-emitting element, the signal generating portion generating a pulse emission signal for controlling a state of the light-emitting element of emitting light and a state of the light-emitting element of not emitting light; and a coaxial cable connecting the light-emitting element and the apparatus body. The coaxial cable has an outer conductor and an inner conductor. The outer conductor is connected to the power supply portion of the light source drive portion or grounded. The inner conductor is connected to the signal generating portion of the light source drive portion.

As described above, in the photoacoustic imaging apparatus according to the aforementioned aspect of the present invention, the coaxial cable is provided to connect the light-emitting element and the apparatus body, the outer conductor of the coaxial cable is connected to the power supply portion of the light source drive portion or grounded, and the inner conductor of the coaxial cable is connected to the signal generating portion of the light source drive portion. By doing so, the likelihood of the occurrence of a reflected wave can be reduced and reduction in the responsivity of a current flowing in the light-emitting element can be suppressed. Further, the likelihood of entry of an electromagnetic wave (noise) from the outside into the inside of the coaxial cable can be reduced. Also, emission of an electromagnetic wave from the inside toward the outside of the coaxial cable can be suppressed. As a result, shortage of the quantity of light emitted from the light-emitting element can be suppressed by reducing the likelihood of entry of an electromagnetic wave, etc. (noise) from outside while suppressing reduction in the responsivity of the current flowing in the light-emitting element, and by suppressing inside-to-outside emission of an electromagnetic wave.

In the photoacoustic imaging apparatus according to the aforementioned aspect, the light source drive portion is preferably configured to generate a flow of a pulsed current of 10A or more in the coaxial cable when the pulse emission signal is generated for placing the light-emitting diode element in the state of emitting light. Flowing a large current as much as 10 A or more in the coaxial cable is not a general use of the coaxial cable. In the present invention, however, a large current of 10A or more is flown as a pulsed current. By doing so, the quantity of light emitted from the light-emitting element can be increased, so that the intensity of the acoustic wave generated from the test object can be increased reliably. The aforementioned flow of the pulsed current of 10A or more in the coaxial cable is not always required to be achieved by flowing the pulsed current of 10 A or more in a single coaxial cable. In the presence of a plurality of coaxial cables, this flow of the pulsed current of 10 A or more can also be achieved if the sum of the values of currents flowing in these coaxial cables is 10 A or more.

In the photoacoustic imaging apparatus according to the aforementioned aspect, the coaxial cable is preferably configured to have a characteristic impedance of 30 $\Omega$ or less. If the characteristic impedance of a cable is high, the responsivity of the current flowing in the light-emitting element is reduced. In this regard, by configuring the coaxial cable in such a manner that the coaxial cable has a characteristic impedance of 30 $\Omega$ or less as in the present invention, reduction in the responsivity of the current flowing in the light-emitting element can be suppressed to a greater degree. This can more reliably suppress shortage of the quantity of light emitted from the light-emitting element caused by reduction in the responsivity of the current flowing in the light-emitting element.

In this case, the coaxial cable is preferably configured to have a characteristic impedance of 15 $\Omega$ or more. The characteristic impedance of the coaxial cable can be reduced by increasing the diameter of the inner conductor of the coaxial cable or reducing the thickness of an insulator provided between the outer conductor and the inner conductor of the coaxial cable. In this case, by configuring the coaxial cable in such a manner that the coaxial cable has a characteristic impedance of 15 Ω or more as in the present invention, the diameter of the inner conductor can be less likely to increase excessively and the thickness of the insulator can be less likely to be reduced excessively. As a result, reduction in the operability of the probe can be suppressed by suppressing excessive increase in the diameter of the inner conductor. Further, reduction in the pressure resistance of the coaxial cable can be suppressed by suppressing excessive reduction in the thickness of the insulator.

In the photoacoustic imaging apparatus according to the aforementioned aspect, the light-emitting element and the apparatus body are preferably connected through a plurality of the coaxial cables. A coaxial cable commonly used has a characteristic impedance of 50 Ω or 75 Ω. As a result of the aforementioned configuration of connecting the light-emitting element and the apparatus body through the coaxial cables, a combined impedance of the coaxial cables can be set easily at a value lower than 50 Ω or 75 Ω by using a commonly-used (general-purposed) coaxial cable, without the need for using a dedicated (customized) coaxial cable.

The photoacoustic imaging apparatus according to the aforementioned aspect preferably further includes: an imaging portion that forms an image of the acoustic wave detected by the detecting portion based on a signal of the acoustic wave; and a signal cable connected to the imaging portion and the detecting portion and transmitting the signal of the acoustic wave, and the coaxial cable and the signal cable are preferably configured to be routed in an integrated state. This configuration prevents separation between the coaxial cable and the signal cable, so that the operability of the cable can be increased, compared to a configuration where the coaxial cable and the signal line are routed separately.

In this case, the photoacoustic imaging apparatus preferably further includes a first shield covering the outside of at least one of the coaxial cable and the signal cable. This configuration allows the first shield to function as a shield against an electromagnetic wave. This makes it possible to shield an electromagnetic wave (noise) to enter at least one of the coaxial cable and the signal cable covered by the first shield, and an electromagnetic wave to be emitted from at least one of the coaxial cable and the signal cable covered by the first shield.

In the aforementioned photoacoustic imaging apparatus where the coaxial cable and the signal cable are configured to be routed in an integrated state, the coaxial cable and the signal cable preferably form a cable group routed in an integrated state, and the photoacoustic imaging apparatus preferably further includes a second shield covering the outside of the cable group. This configuration allows the second shield to function as a shield against an electromagnetic wave. This makes it possible to shield an electromagnetic wave (noise) to enter the cable group from outside and an electromagnetic wave to be emitted to the outside of the cable group.

In the photoacoustic imaging apparatus according to the aforementioned aspect, the outer conductor of the coaxial cable is preferably connected to the power supply portion of the light source drive portion, and the inner conductor of the coaxial cable is preferably connected to the signal generating portion. If the outer conductor of the coaxial cable is configured to be grounded and the inner conductor of the coaxial cable is configured to be connected to the signal generating portion, a power supply portion capable of applying a negative voltage to be connected to the signal generating portion should be provided. Providing a power supply portion capable of applying a negative voltage generally makes the configuration of the photoacoustic imaging apparatus more complicated than providing the power supply portion capable of applying a positive voltage. In this regard, by employing the aforementioned configuration where the outer conductor of the coaxial cable is connected to the power supply portion of the light source drive portion and the inner conductor of the coaxial cable is connected to the signal generating portion, the need for providing a power supply portion capable of applying a negative voltage is eliminated. As a result, while complication of the photoacoustic imaging apparatus is suppressed, shortage of the quantity of light emitted from the light-emitting element can be suppressed.

In the photoacoustic imaging apparatus according to the aforementioned aspect, the coaxial cable is preferably configured to have a conductor resistance of 0.5 Ω/m or less. This configuration can reduce power loss occurring in the coaxial cable due to the conductor resistance, compared to the coaxial cable configured to have a conductor resistance higher than 0.5 Ω/m.

The photoacoustic imaging apparatus according to the aforementioned aspect preferably further includes: a light source portion including the light-emitting element; a substrate having a first surface and a second surface opposite the first surface, the light source portion being arranged on the first surface, a wire being arranged on the first surface or the second surface; and an electromagnetic wave absorption layer provided to cover the wire from a place adjacent to the second surface of the substrate. It is probable that the likelihood of entry of an electromagnetic wave, etc. (noise) from outside will not be reduced sufficiently and inside-to-outside emission of an electromagnetic wave will not be suppressed sufficiently for a structure other than the coaxial cable, specifically, the light source portion including the light-emitting element. In this regard, in the present invention, the photoacoustic imaging apparatus includes the electromagnetic wave absorption layer covering the wire from a place adjacent to the second surface of the substrate. This allows the electromagnetic wave absorption layer to absorb an electromagnetic wave generated from the light source portion and the wire connected to the light source portion to travel toward the detecting portion near the light source portion. Thus, the detecting portion is allowed to be less likely to detect an electromagnetic wave, making it possible to reduce the likelihood of inclusion of noise in an image to be formed by the photoacoustic imaging apparatus.

In this case, the electromagnetic wave absorption layer preferably includes a substrate exposing portion for exposing the second surface of the substrate, and the photoacoustic imaging apparatus preferably further includes a heat conducting portion for dissipating heat from the substrate. The heat conducting portion is arranged to contact the second surface of the substrate through the substrate exposing portion of the electromagnetic wave absorption layer. In this configuration, heat generated from the light source portion can be dissipated effectively by the heat conducting portion through the second surface of the substrate. As a result, the lifetime of the light source portion can be extended.

The aforementioned photoacoustic imaging apparatus including the heat conducting portion preferably further includes a housing accommodating detecting portion, and the heat conducting portion is preferably configured to contact the second surface of the substrate at one end of the heat conducting portion and to contact the housing at an opposite end of the heat conducting portion. This configuration allows the heat conducting portion to transfer heat generated from the light source portion toward the housing. As a result, the heat generated from the light source portion can be dissipated more effectively.

In the aforementioned photoacoustic imaging apparatus including the housing accommodating the detecting portion, the housing preferably includes a heat dissipating portion, and the heat conducting portion is preferably configured to contact the heat dissipating portion at the opposite end of the heat conducting portion. This configuration allows the heat dissipating portion adjacent to the opposite end of the heat conducting portion to still more effectively dissipate heat generated from the light source portion.

In the aforementioned photoacoustic imaging apparatus including the electromagnetic wave absorption layer, an insulating member is preferably provided between the second surface of the substrate and the electromagnetic wave absorption layer. This configuration allows the insulating layer to function to increase an insulation breakdown voltage. As a result, a high voltage can be applied to the light source portion, so that the intensity of light emitted from the light source portion can be increased.

The photoacoustic imaging apparatus according to the aforementioned aspect preferably further includes a light source portion including the light-emitting element, and the light source portion and the detecting portion are preferably arranged adjacent to each other. Light from the light source portion and the acoustic wave from the test object are attenuated more with a greater distance of propagation. In view of this point, in the present invention, the light source portion and the detecting portion are arranged adjacent to each other. This makes it possible to separate the light source portion, the detecting portion, and the test object by relatively small distances. In this way, the detecting portion is allowed to detect the acoustic wave efficiently while attenuation of the light from the light source portion and that of the acoustic wave from the test object are suppressed.

In the photoacoustic imaging apparatus according to the aforementioned aspect, the light-emitting element preferably includes a plurality of light-emitting elements, and the light-emitting elements are preferably arranged in a linear pattern. In this configuration, even if the quantity of light emitted from each of the light-emitting elements is small, the presence of the light-emitting elements arranged in a linear pattern allows the light-emitting elements as a whole to produce a light quantity sufficient for imaging the acoustic wave.

In the photoacoustic imaging apparatus according to the aforementioned aspect, the light-emitting element is preferably formed of a light-emitting diode element. The light-emitting diode element has lower directivity than a light-emitting element for emitting a laser beam. Thus, in this configuration, even on the occurrence of position shift, a range of light emission is comparatively unlikely to change. This eliminates the need for exact alignment (positioning) of an optical member and eliminates the need for an optical surface plate or a strong housing for suppressing characteristic fluctuation due to vibration of an optical system, unlike in the case of using a light-emitting element for emitting a laser beam. As a result, size increase of the photoacoustic imaging apparatus and complication of the configuration of the photoacoustic imaging apparatus can be suppressed by eliminating the need for exact alignment of an optical member and eliminating the need for an optical surface plate or a strong housing. Further, the quantity of light emitted from the single light-emitting diode element is smaller than that of light emitted from a light-emitting element for emitting a laser beam, for example. Thus, the light-emitting diode element is preferably arranged near the detecting portion. In this regard, as described above, the coaxial cable is provided to connect the probe and the apparatus body. As a result, shortage of the quantity of light emitted from the light-emitting diode element can be suppressed by reducing the likelihood of entry of an electromagnetic wave, etc. (noise) from outside while suppressing reduction in the responsivity of the current flowing in the light-emitting diode element, and by suppressing inside-to-outside emission of an electromagnetic wave more effectively.

In the photoacoustic imaging apparatus according to the aforementioned aspect, the light-emitting element is preferably formed of a semiconductor laser element. The semiconductor laser element is capable of emitting a laser beam of relatively high directivity, compared to the light-emitting diode element. Thus, in this configuration, much of the beam from the semiconductor laser element can be applied reliably to the test object.

In the photoacoustic imaging apparatus according to the aforementioned aspect, the light-emitting element is preferably formed of an organic light-emitting diode element. In this configuration, using the organic light-emitting diode element that can be reduced easily in thickness, the probe portion including the organic light-emitting diode element can be reduced easily in size.

Advantageous Effects of Invention

As described above, the present invention is capable of suppressing shortage of the quantity of light emitted from the light-emitting element by reducing the likelihood of entry of an electromagnetic wave, etc. (noise) from outside while suppressing reduction in the responsivity of the current flowing in the light-emitting element, and by suppressing inside-to-outside emission of an electromagnetic wave.

Brief Description of Drawings

FIG. 1 is a perspective view showing the overall configuration of a photoacoustic imaging apparatus according to a first embodiment of the present invention.

FIG. 2 is a perspective view showing the configuration of a probe according to the first embodiment of the present invention.

FIG. 3 is a view for explaining the configuration of a coaxial cable according to the first embodiment of the present invention.

FIG. 4 is a block diagram showing the overall configuration of the photoacoustic imaging apparatus according to the first embodiment of the present invention.

FIG. 5 is a view for explaining a result of experiment conducted about the operation of the photoacoustic imaging apparatus according to the first embodiment of the present invention.

FIG. 6 is a view for explaining a result of experiment conducted about the operation of a photoacoustic imaging apparatus not using the coaxial cable.

FIG. 7 is a view for explaining a relationship between the characteristic impedance of a coaxial cable and the response time of a current flowing in a light-emitting diode established in a second embodiment of the present invention.

FIG. 8 is a block diagram showing a part of the configuration of a photoacoustic imaging apparatus according to a third embodiment of the present invention.

FIG. 9 is a perspective view showing the overall configuration of a photoacoustic imaging apparatus according to a fourth embodiment of the present invention.

FIG. 10 is a sectional view showing the configuration of a cable of the photoacoustic imaging apparatus according to the fourth embodiment of the present invention.

FIG. 11 is a perspective view showing the configuration of a probe according to the fourth embodiment of the present invention.

FIG. 12 is a sectional view showing the configuration of a cable of a photoacoustic imaging apparatus according to a fifth embodiment of the present invention.

FIG. 13 is a perspective view showing the overall configuration of a photoacoustic imaging apparatus according to a sixth embodiment of the present invention.

FIG. 14 is a sectional view of a second housing of the photoacoustic imaging apparatus according to the sixth embodiment of the present invention.

FIG. 15 is a view showing a first surface of a substrate of the photoacoustic imaging apparatus according to the sixth embodiment of the present invention.

FIG. 16 is a view showing a state where an electromagnetic wave absorption layer is provided to a second surface of the substrate of the photoacoustic imaging apparatus according to the sixth embodiment of the present invention.

FIG. 17 is a sectional view of a first housing of the photoacoustic imaging apparatus according to the sixth embodiment of the present invention.

FIG. 18 is a block diagram of the photoacoustic imaging apparatus according to the sixth embodiment of the present invention.

FIG. 19 is a schematic view for explaining an image of a detection target formed by a photoacoustic imaging apparatus without an electromagnetic wave absorption layer.

FIG. 20 is a schematic view for explaining an image of a detection target formed by the photoacoustic imaging apparatus according to the sixth embodiment of the present invention.

FIG. 21 is a sectional view of a second housing of a photoacoustic imaging apparatus according to a seventh embodiment of the present invention.

FIG. 22 is a perspective view showing the overall configuration of a photoacoustic imaging apparatus according to an eighth embodiment of the present invention.

FIG. 23 is a perspective view showing the configuration of an apparatus body according to a first modification of the first embodiment of the present invention.

FIG. 24 is a block diagram showing the configuration of a light source drive portion according to a second modification of the first embodiment of the present invention.

FIG. 25 is a view showing the configuration of an illumination portion according to each of a third modification and a fourth modification of the first embodiment of the present invention.

FIG. 26 is a sectional view showing the configuration of a cable according to a fifth modification of the fourth and fifth embodiments of the present invention.

FIG. 27 is a sectional view of a second housing of a photoacoustic imaging apparatus according to a sixth modification of the sixth embodiment of the present invention.

Description of Embodiments

Embodiments of the present invention will be described below based on the drawings.

(First Embodiment)

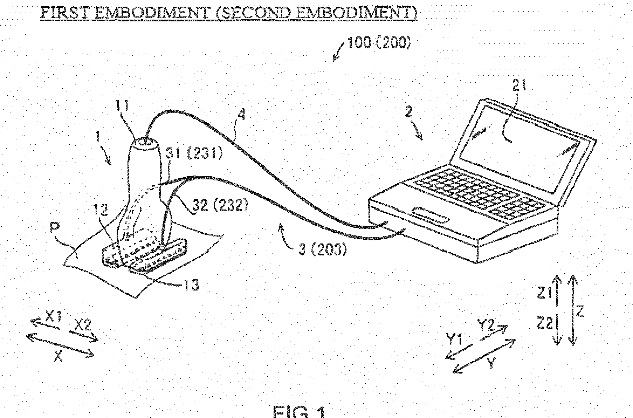

The configuration of a photoacoustic imaging apparatus 100 according to a first embodiment of the present invention will be described by referring to FIGS. 1 to 4.

As shown in FIG. 1, the photoacoustic imaging apparatus 100 according to the first embodiment of the present invention includes a probe 1 and an apparatus body 2. The photoacoustic imaging apparatus 100 further includes a coaxial cable 3 and a signal cable 4.

The coaxial cable 3 and the signal cable 4 are each configured to have a length about 2 m, for example, and to connect the probe 1 and the apparatus body 2.

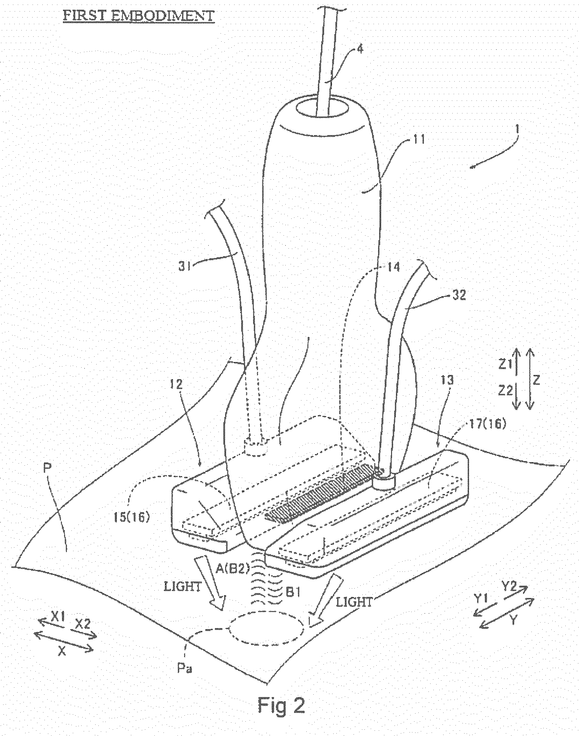

The probe 1 is configured to move over a surface of a test object P (a surface of a human body, for example) while being grasped by an operator. The coaxial cable 3 is configured to transfer power from the apparatus body 2 to the probe 1. The probe 1 is configured to be capable of generating light in response to the power acquired through the coaxial cable 3 and emitting the light to the test object P. As shown in FIG. 2, the probe 1 is configured to detect an acoustic wave A and an ultrasonic wave B2 traveling from the inside of the test object P, and to transmit the received acoustic wave A and ultrasonic wave B2 as received signals to the apparatus body 2 through the signal cable 4.

As shown in FIG. 1, the apparatus body 2 is configured to form an image by processing the received signals detected by the probe 1. The apparatus body 2 includes an image display portion 21. The image display portion 21 is formed of a liquid crystal panel, etc., and is configured to display the image acquired from the apparatus body 2.

As shown in FIG 2, the probe 1 includes a probe body 11, an illumination portion 12, and an illumination portion 13. More specifically, the probe body 11 is formed into a streamline shape. The illumination portion 12 is arranged near the tip of the probe body 11 (closer to a direction of an arrow Z2) and arranged closer to a direction of an arrow X1. The illumination portion 13 is arranged near the tip of the probe body 11 (closer to the direction of the arrow Z2) and arranged closer to a direction of an arrow X2. The illumination portions 12 and 13 are arranged so as to sandwich the probe body 11 from opposite sides of the direction X. An acoustic wave detecting portion 14 is arranged at the tip of the probe body 11. Specifically, the illumination portions 12 and 13 are arranged near the acoustic wave detecting portion 14. The acoustic wave detecting portion 14 is an example of a "detecting portion" according to the present invention.

The illumination portion 12 includes a light source portion 15. The light source portion 15 includes a plurality of (108, for example) light-emitting diode elements 16 capable of emitting light to the test object P. Like the illumination portion 12, the illumination portion 13 includes a light source portion 17 including a plurality of light-emitting diode elements 16. The light-emitting diode elements 16 are arranged in an array pattern (in a linear pattern). The light-emitting diode elements 16 arranged in an array pattern are together configured as a surface light source. The light-emitting diode elements 16 are an example of a "light-emitting element" according to the present invention.

The coaxial cable 3 includes a coaxial cable 31 and a coaxial cable 32. The coaxial cable 31 is connected to the illumination portion 12 to be closer to a direction of an arrow Z1. The coaxial cable 32 is connected to the illumination portion 13 to be closer to the direction of the arrow Z1.

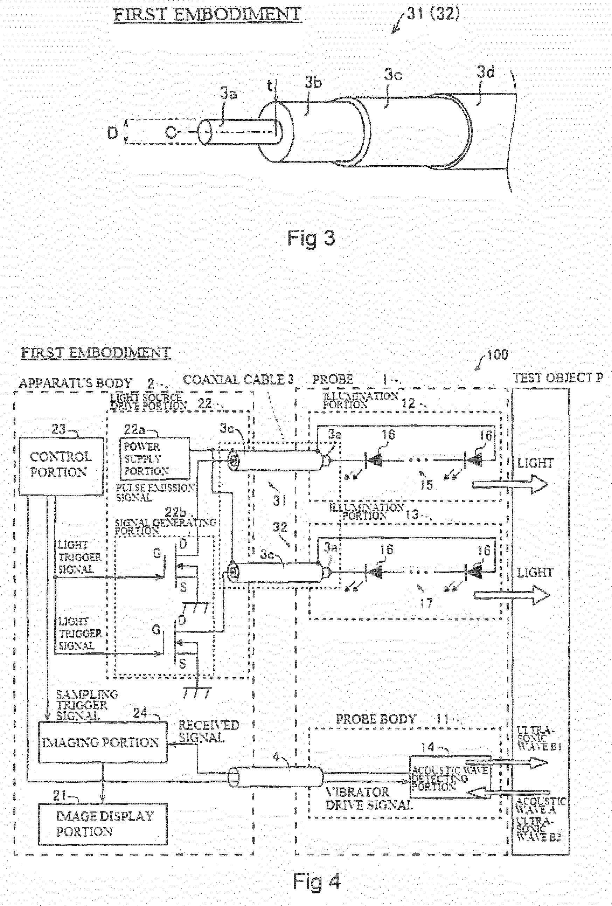

As shown in FIG. 3, the coaxial cable 31 has a size such as AWG 20 (conforming to UL standards), AWG 30, AWG 36, or AWG 40, for example. The coaxial cable 31 is formed of an inner conductor 3a, an insulator 3b, an outer conductor 3c, and a jacket 3d.

The inner conductor 3a is arranged at a central area C of the coaxial cable 31. For example, the inner conductor 3a is formed of an annealed copper wire, a silver-plated annealed copper wire, a tinned copper alloy wire, or a tinned annealed copper wire, for example. The inner conductor 3a is formed of a single wire or a plurality of (seven, for example) twisted wires. The inner conductor 3a is configured in such a manner that the outer diameter D of the inner conductor 3a (if the inner conductor 3a is formed of a plurality of twisted wires, the outer diameter of the entire twisted wires) is from 0.26 to 0.30 mm, for example.

The insulator 3b is provided to cover an outer peripheral surface of the inner conductor 3a. The insulator 3b is made of polyethylene, FEP tetrafluoroethylene-hexafluoropropylene copolymer), or PFA (perfluoroalkoxy fluorocarbon resin), for example. The insulator 3b is configured to have a thickness t from 0.08 to 0.40 mm.

The outer conductor 3c is provided to cover an outer peripheral surface of the insulator 3b and is configured to have the function of shielding the inner conductor 3a from an electromagnetic wave (noise) coming from the outside of the outer conductor 3c. The outer conductor 3c is also configured to fulfill a function as a shield against an electromagnetic wave (noise) to travel outward from the inner conductor 3a. The outer conductor 3c is formed of an annealed copper wire, a tinned copper alloy wire, or a tinned annealed copper wire, for example. The outer conductor 3c is formed of a braided or served elemental wire of a diameter from 0.03 to 0.08 mm, for example.

The jacket 3d is provided to cover an outer peripheral surface of the outer conductor 3c. For example, the jacket 3d is made of FEP, PVC (polyvinyl chloride), PFA, or PET (polyethylene terephthalate), for example.

With the above-described configuration, the coaxial cable 31 is configured to have a conductor resistance of about 1.0 $\Omega$/2m or less and a characteristic impedance from 22 to 75 $\Omega$.

As shown in FIG. 4, the apparatus body 2 includes a light source drive portion 22 and a control portion 23. The light source drive portion 22 is configured to acquire power from an external power supply portion (not shown in the drawings) and to supply the acquired power to the light-emitting diode element 16 through the coaxial cable 3. The control portion 23 includes a CPU (central processing unit), etc., and is configured to control the photoacoustic imaging apparatus 100 in its entirety by transmitting a control signal to each structure.

The light source drive portion 22 includes a power supply portion 22a and a signal generating portion 22b.

In the first embodiment, regarding the coaxial cables 31 and 32, the outer conductor 3c of each of the coaxial cables 31 and 32 is connected to the power supply portion 22a of the light source drive portion 22. The inner conductor 3a of each of the coaxial cables 31 and 32 is connected to the signal generating portion 22b of the light source drive portion 22. The light source drive portion 22 is configured to generate a flow of a pulsed current of 10A or more in each of the coaxial cables 31 and 32 when a pulse emission signal is generated for placing the light-emitting diode element 16 in a state of emitting light.

More specifically, the power supply portion 22a is connected to the outer conductor 3c of each of the coaxial cables 31 and 32. The power supply portion 22a includes a DC/DC converter, for example, and is configured to apply an intended voltage (about 200 V, for example). The outer conductor 3c of each of the coaxial cables 31 and 32 is connected to the anode of the light-emitting diode element 16 and is configured to apply the intended voltage to the anode.

The signal generating portion 22b includes two FETs (field effect transistors), for example. The drain of one of the two FETs is connected to the inner conductor 3a of the coaxial cable 31. The drain of the other of the two FETs is connected to the inner conductor 3a of the coaxial cable 32. The inner conductor 3a of each of the coaxial cables 31 and 32 is connected to the cathode of the light-emitting diode element 16. The source of each of the FETs in the signal generating portion 22b is grounded.

The signal generating portion 22b is configured to be capable of generating a flow of a pulsed current (15 A (10A or more) in terms of a peak current) from the anode toward the cathode of the light-emitting diode element 16 by generating a pulse emission signal if the FETs in the signal generating portion 22b are turned on by input of a pulsed light trigger signal from the control portion 23 to the gates. The light-emitting diode element 16 is configured to emit pulsed light responsive to the pulsed current to the test object P. The light source drive portion 22 and the control portion 23 are configured in such a manner that the pulsed light has a pulse width about 150 ns, for example. Generating the pulse emission signal means reducing a voltage at the cathode of the light-emitting diode element 16.

As shown in FIG 2, light emitted from the probe 1 to the test object P is absorbed by a detection target Pa (hemoglobin, for example) in the test object P. Then, the detection target Pa expands and contracts (the detection target Pa in an expanded state restores its original size) according to the emission intensity of the pulsed light (the quantity of the absorbed light), thereby generating the acoustic wave A from the detection target Pa (test object P). In this description, for the convenience of explanation, an ultrasonic wave generated by absorption of light by the detection target Pa in the test object P will be called the "acoustic wave A," an ultrasonic wave generated by the acoustic wave detecting portion 14 and reflected on the test object P will be called the "ultrasonic wave B2," and the acoustic wave A and the ultrasonic wave B2 will be described distinctively.

The acoustic wave detecting portion 14 includes an ultrasonic vibrator having 128 channels (not shown in the drawings). The ultrasonic vibrator of the acoustic wave detecting portion 14 is formed of a piezoelectric element (made of lead zirconate titanate (PZT), for example), and is configured to vibrate to generate a voltage (received signal) if the aforementioned acoustic wave A is acquired. The acoustic wave detecting portion 14 is configured to transmit the acquired received signal to an imaging portion 24 (see FIG. 4) described later.

The ultrasonic vibrator of the acoustic wave detecting portion 14 is configured to be capable of generating the ultrasonic wave B1 by vibrating at a frequency responsive to a vibrator drive signal from the control portion 23. The ultrasonic vibrator is configured to apply the ultrasonic wave B1 to the test object P.

As shown in FIG. 2, the ultrasonic wave B1 generated by the acoustic wave detecting portion 14 is reflected on a substance having a high acoustic impedance (detection target Pa) in the test object P. The ultrasonic wave B2 (resulting from reflection of the ultrasonic wave B1) is acquired by the acoustic wave detecting portion 14.

Like in the case of acquiring the acoustic wave A, if the ultrasonic wave B2 is acquired, the acoustic wave detecting portion 14 is configured to transmit the received signal to the imaging portion 24. The photoacoustic imaging apparatus 100 is configured in such a manner that a period when the acoustic wave detecting portion 14 acquires the acoustic wave A and a period when the acoustic wave detecting portion 14 acquires the ultrasonic wave B2 do not overlap. By doing so, the photoacoustic imaging apparatus 100 is configured to be capable of making a distinction between the acoustic wave A and the ultrasonic wave B2.

As shown in FIG 4, the apparatus body 2 includes the imaging portion 24. The imaging portion 24 is configured to acquire a sampling trigger signal synchronized with a light trigger signal from the control portion 23 and to acquire a received signal from the acoustic wave detecting portion 14. The imaging portion 24 is configured to form a tomographic image responsive to the acoustic wave A and a tomographic image responsive to the ultrasonic wave B2 based on the acquired sampling trigger signal and the acquired received signal, and to execute processing of combining the tomographic images. The imaging portion 24 is configured to output a composite image to the image display portion 21.

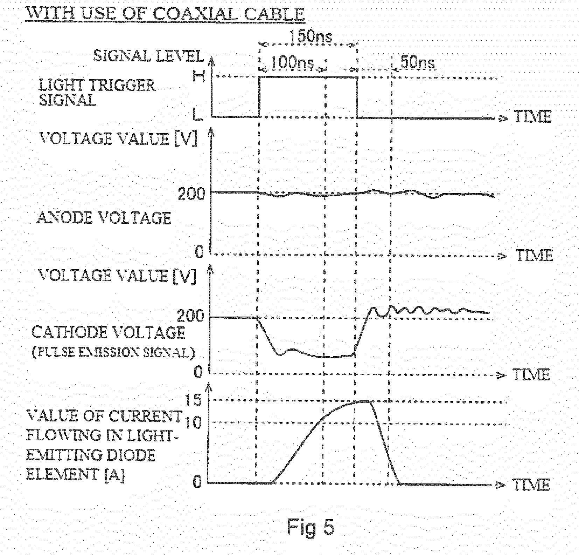

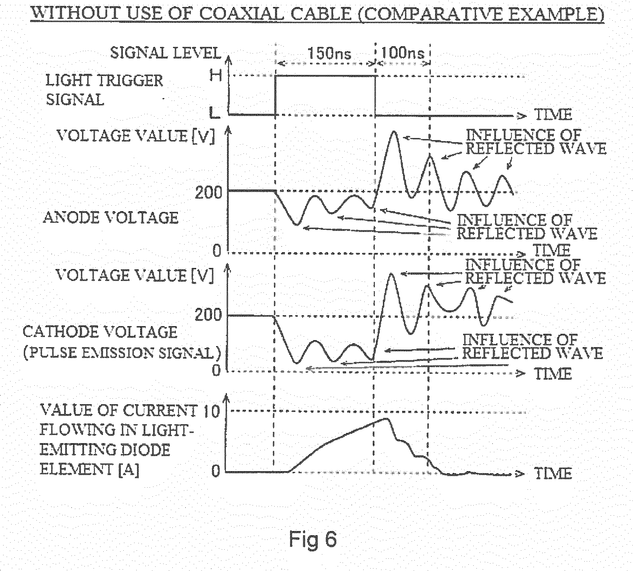

Described next by referring to FIGS. 5 and 6 is experiment conducted to make a comparison of responsivity of a current flowing in the light-emitting diode element 16 between the case of using the coaxial cable 3 (first embodiment) in the photoacoustic imaging apparatus 100 according to the first embodiment and the case of not using the coaxial cable 3 (the case of using a twisted pair cable) (Comparative Example).

In this experiment, a light trigger signal having a pulse width of 150 ns was first input to the photoacoustic imaging apparatus 100 according to the first embodiment using the coaxial cable 3 (see FIG. 5) and to a photoacoustic imaging apparatus using the twisted pair cable (see FIG. 6). Then, the waveform of the anode voltage of the light-emitting diode element 16, the waveform of the cathode voltage of the light-emitting diode element 16, and the waveform of the value of a current flowing in the light-emitting diode element 16 were measured. Response time was measured by acquiring the waveform of the value of the current flowing in the light-emitting diode element 16.

Measurements were obtained in the photoacoustic imaging apparatus using the twisted pair cable by connecting one of conductive wires of a twisted pair to a power supply portion and the anode of a light-emitting diode element and by connecting the other of the conductive wires of the twisted pair to a signal generating portion and the cathode of the light-emitting diode element.

As shown in FIG. 5, in a period when a light trigger signal was at a signal level H (high) (150 ns), the waveform of the anode voltage shows that the anode voltage was constant at substantially 200 V in the photoacoustic imaging apparatus 100 using the coaxial cable 3. By contrast, as shown in FIG. 6, in the photoacoustic imaging apparatus using the twisted pair cable, the waveform of the anode voltage shows that the anode voltage varied while a reflected wave of a cycle of 50 ns and an amplitude (a voltage value between a maximum and a minimum) of 80 V was generated.

As shown in FIG. 5, in the photoacoustic imaging apparatus 100 using the coaxial cable 3, the waveform of the cathode voltage (the waveform of a pulse emission signal) shows that the cathode voltage was constant at substantially 60 V. By contrast, as shown in FIG. 6, the waveform of the cathode voltage shows that the cathode voltage varied while a reflected wave was generated in the photoacoustic imaging apparatus using the twisted pair cable.

As shown in FIG. 5, the value of the current flowing in the light-emitting diode element 16 reached substantially 15 A (10A or more) after elapse of 100 ns after the light trigger signal was placed at a signal level H in the photoacoustic imaging apparatus 100 using the coaxial cable 3. By contrast, as shown in FIG 6, the value of the current flowing in the light-emitting diode element 16 did not reach 10A after the light trigger signal was placed at a signal level H in the photoacoustic imaging apparatus using the twisted pair cable.

As shown in FIG. 5, the waveform of the anode voltage and the waveform of the cathode voltage show that the anode voltage and the cathode voltage were constant at substantially 200 V after the light trigger signal was placed at a signal level L (low) in the photoacoustic imaging apparatus 100 using the coaxial cable 3. By contrast, as shown in FIG. 6, the waveform of the anode voltage and the waveform of the cathode voltage show that the anode voltage and the cathode voltage varied while a reflected wave of an amplitude of 220 V was generated in the photoacoustic imaging apparatus using the twisted pair cable.

As shown in FIG. 5, the value of the current flowing in the light-emitting diode element 16 became substantially zero after elapse of 50 ns after the light trigger signal was placed at a signal level L in the photoacoustic imaging apparatus 100 using the coaxial cable 3. By contrast, as shown in FIG. 6, the value of the current flowing in the light-emitting diode element 16 became substantially zero after elapse of 100 ns after the light trigger signal was placed at a signal level L in the photoacoustic imaging apparatus using the twisted pair cable.

As understood from the results given above, in the photoacoustic imaging apparatus 100 using the coaxial cable 3 (first embodiment), the response time of the current flowing in the light-emitting diode element 16 is 150 ns (100 ns + 50 ns), whereas in the photoacoustic imaging apparatus using the twisted pair cable (Comparative Example), the response time of the current flowing in the light-emitting diode element 16 is at least 250 ns or more. As also understood from the results given above, in the photoacoustic imaging apparatus 100 using the coaxial cable 3, the value of the current flowing in the light-emitting diode element 16 reaches 15A, whereas in the photoacoustic imaging apparatus using the twisted pair cable, the value of this current is less than 10 A (up to about 9 A). Specifically, the photoacoustic imaging apparatus 100 using the coaxial cable 3 is determined to be capable of producing a steep waveform of light (the waveform of the value of the current flowing in the light-emitting diode element 16) and increasing a light quantity, compared to the photoacoustic imaging apparatus using the twisted pair cable.

The first embodiment achieves the following effect.

As described above, in the first embodiment, the coaxial cable 3 is provided to connect the probe 1 (light-emitting diode element 16) and the apparatus body 2. The outer conductor 3c of the coaxial cable 3 is connected to the power supply portion 22a of the light source drive portion 22 and the inner conductor 3a of the coaxial cable 3 is connected to the signal generating portion 22b of the light source drive portion 22. By doing so, the likelihood of the occurrence of a reflected wave can be reduced and reduction in the responsivity of a current flowing in the light-emitting diode element 16 can be suppressed. Further, the likelihood of entry of an electromagnetic wave (noise) from the outside into the inside of the coaxial cable 3 can be reduced. As a result, shortage of the quantity of light emitted from the light-emitting diode element 16 can be suppressed by reducing the likelihood of entry of an electromagnetic wave, etc. (noise) from outside while suppressing reduction in the responsivity of the current flowing in the light-emitting diode element 16, and by suppressing inside-to-outside emission of an electromagnetic wave.

As described above, in the first embodiment, the light source drive portion 22 is configured to generate a flow of a pulsed current of 10A or more in the coaxial cable 3 when a pulse emission signal is generated for placing the light-emitting diode element 16 in a state of emitting light. Flowing a large current as much as 10 A or more in the coaxial cable 3 is not a general use of the coaxial cable 3. In the present invention, however, a large current of 10A or more is flown as a pulsed current. By doing so, the quantity of light emitted from the light-emitting diode element 16 can be increased, so that the intensity of the acoustic wave A generated from the test object P can be increased reliably.

As described above, in the first embodiment, the outer conductor 3c of the coaxial cable 3 is connected to the power supply portion 22a of the light source drive portion 22 and the inner conductor 3a of the coaxial cable 3 is connected to the signal generating portion 22b. If the outer conductor 3c of the coaxial cable 3 is configured to be grounded and the inner conductor 3a of the coaxial cable 3 is configured to be connected to the signal generating portion 22b, a power supply portion capable of applying a negative voltage to be connected to the signal generating portion 22b should be provided. Providing a power supply portion capable of applying a negative voltage generally makes the configuration of the photoacoustic imaging apparatus 100 more complicated than providing the power supply portion 22a capable of applying a positive voltage. In this regard, the aforementioned configuration of the first embodiment eliminates the need for providing a power supply portion capable of applying a negative voltage. As a result, while complication of the photoacoustic imaging apparatus 100 is suppressed, shortage of the quantity of light emitted from the light-emitting diode element 16 can be suppressed.

As described above, in the first embodiment, the light-emitting diode element 16 is provided in each of the light source portions 15 and 17. The light-emitting diode element 16 has lower directivity than a light-emitting element for emitting a laser beam. Thus, even on the occurrence of position shift, a range of light emission is comparatively unlikely to change. This eliminates the need for exact alignment (positioning) of an optical member and eliminates the need for an optical surface plate or a strong housing for suppressing characteristic fluctuation due to vibration of an optical system, unlike in the case of using a light-emitting element for emitting a laser beam. As a result, size increase of the photoacoustic imaging apparatus 100 and complication of the configuration of the photoacoustic imaging apparatus 100 can be suppressed by eliminating the need for exact alignment of an optical member and eliminating the need for an optical surface plate or a strong housing. Further, the quantity of light emitted from the single light-emitting diode element 16 is smaller than that of light emitted from a light-emitting element for emitting a laser beam, for example. Thus, the light-emitting diode element 16 is preferably arranged near the acoustic wave detecting portion 14. In this regard, in the first embodiment, the coaxial cable 3 is provided to connect the probe 1 (light-emitting diode element 16) and the apparatus body 2. As a result, shortage of the quantity of light emitted from the light-emitting diode element 16 can be suppressed by reducing the likelihood of entry of an electromagnetic wave, etc. (noise) from outside while suppressing reduction in the responsivity of a current flowing in the light-emitting diode element 16 more effectively.

As described above, in the first embodiment, the coaxial cable 3 is configured to have a conductor resistance of 0.5 $\Omega$/m or less (1.0 $\Omega$/2m). This can reduce power loss occurring in the coaxial cable 3 due to the conductor resistance, compared to the coaxial cable 3 configured to have a conductor resistance higher than 0.5 $\Omega$/m.

As descried above, in the first embodiment, the light source portions 15 and 17 each including the light-emitting diode element 16 are further provided. The light source portion 15 (illumination portion 12) and the acoustic wave detecting portion 14, and the light source portion 17 (illumination portion 13) and the acoustic wave detecting portion 14 are arranged adjacent to each other. Light from each of the light source portions 15 and 17 and the acoustic wave A from the test object P are attenuated more with a greater distance of propagation. In view of this point, in the first embodiment, the light source portion 15 and the acoustic wave detecting portion 14, and the light source portion 17 and the acoustic wave detecting portion 14 are arranged adjacent to each other. This makes it possible to separate the light source portion 15, the acoustic wave detecting portion 14, and the test object P by relatively small distances, while separating the light source portion 17, the acoustic wave detecting portion 14, and the test object P by relatively small distances. In this way, the acoustic wave detecting portion 14 is allowed to detect the acoustic wave A efficiently while attenuation of light from each of the light source portions 15 and 17 and that of the acoustic wave A from the test object P are suppressed.

As described above, in the first embodiment, the light-emitting diode elements 16 are arranged in a linear pattern (array pattern). By doing so, even if the quantity of light emitted from each of the light-emitting diode elements 16 is small, the presence of the light-emitting diode elements 16 arranged in a linear pattern allows the light source portions 15 and 17 as a whole to produce a light quantity sufficient for imaging the acoustic wave A.

(Second Embodiment)

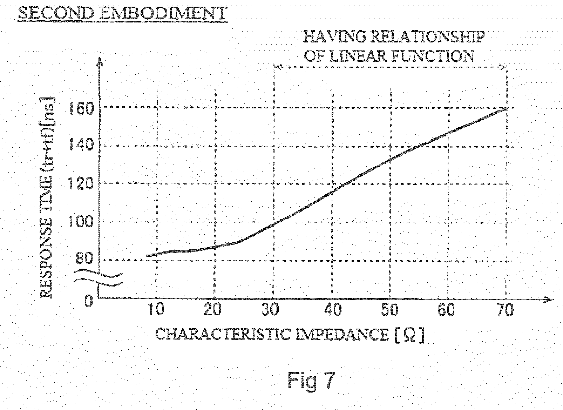

The configuration of a photoacoustic imaging apparatus 200 according to a second embodiment of the present invention will be described next by referring to FIGS. 1 and 7. In the second embodiment, the photoacoustic imaging apparatus includes a coaxial cable having a characteristic impedance from 15 to 30 $\Omega$.

As shown in FIG. 1, the photoacoustic imaging apparatus 200 according to the second embodiment includes a coaxial cable 203. The coaxial cable 203 includes a coaxial cable 231 and a coaxial cable 232.

In the second embodiment, the coaxial cables 231 and 232 are each configured to have a characteristic impedance from 15 to 30 $\Omega$.

FIG. 7 shows a relationship between the characteristic impedance of each of the coaxial cables 231 and 232 and the response time of a current flowing in the light-emitting diode element 16. The response time (tr + tf) is the sum of time tr from acquisition of a pulse emission signal by the light-emitting diode element 16 to a moment when a current value becomes a substantially peak value, and time tf from stop of the pulse emission signal to a moment when the current value becomes substantially zero.

If the characteristic impedance of the coaxial cable 203 is higher than 30 $\Omega$, a substantially linear function is established as a relationship between the characteristic impedance and the response time. Specifically, the characteristic impedance of the coaxial cable 203 and the response time of a current flowing in the light-emitting diode element 16 are related in such a manner that the response time increases with increase in the characteristic impedance.

If the characteristic impedance of the coaxial cable 203 is 30 $\Omega$ or less, the response time is relatively constant (from 80 to 100 ns) with respect to the characteristic impedance. Similar measurement was made in a state where the impedances of cables were substantially uninfluential produced by arranging the light source drive portion 22 and the light source portion 15 close to each other, arranging the light source drive portion 22 and the light source portion 17 close to each other, and setting the lengths of the cables at 5 cm. The response time obtained by this measurement is 100 ns. This shows that, by configuring the coaxial cable 203 in such a manner that the coaxial cable 203 has a characteristic impedance of 30 $\Omega$ or less, the response time of the current flowing in the light-emitting diode element 16 can be 100 ns or less.

The characteristic impedance Z of the coaxial cable 203 (coaxial cables 231 and 232) can be expressed by the following formula (1) using the inductance L and the capacitance C of the coaxial cable 203:

$$Z = \sqrt{\left(\frac{L}{C}\right)} \quad \dots (1)$$

As understood from the foregoing formula (1), the inductance L of the coaxial cable 203 can be reduced by increasing the outer diameter D of the inner conductor 3a (see FIG. 3). Thus, the characteristic impedance Z can be reduced by increasing the outer diameter D of the inner conductor 3a. However, increasing the outer diameter D of the inner conductor 3a excessively leads to size increase of the coaxial cable 203, which is not preferable in terms of handling of the probe 1. For example, the outer diameter D of the inner conductor 3a is preferably about 0.3 mm (about AWG 30).

As understood from the foregoing formula (1), the capacitance C of the coaxial cable 203 can be increased by reducing the thickness t of the insulator 3b. Thus, the characteristic impedance Z can be reduced by reducing the thickness t of the insulator 3b. However, reducing the thickness t of the insulator 3b excessively leads to shortage of the pressure resistance (withstand voltage) of the coaxial cable 203. If the size of the coaxial cable 203 is AWG 30, for example, the pressure resistance of the coaxial cable 203 can be maintained at 250 V by configuring the insulator 3b in such a manner that the insulator 3b has the thickness t with which the characteristic impedance of the coaxial cable 203 becomes 15 $\Omega$ or more.

The configuration of the photoacoustic imaging apparatus 200 according to the second embodiment is the same in the other respects as the photoacoustic imaging apparatus 100 according to the first embodiment.

The second embodiment achieves the following effect.

As described above, in the second embodiment, the coaxial cable 203 is configured to have a characteristic impedance of 30 Ω or less. By doing so, increase in the response time (responsivity) of a current flowing in the light-emitting diode element 16 can be suppressed more than configuring the coaxial cable 203 in such a manner that the coaxial cable 203 has a characteristic impedance higher than 30 Ω. This can more reliably suppress shortage of the quantity of light emitted from the light-emitting diode element 16 caused by reduction in the responsivity of the current flowing in the light-emitting diode element 16.

As described above, in the second embodiment, the coaxial cable 203 is configured to have a characteristic impedance of 15 Ω or more. By doing so, the outer diameter D of the inner conductor 3a can be less likely to increase excessively and the thickness t of the insulator 3b can be less likely to be reduced excessively than configuring the coaxial cable 203 in such a manner that the coaxial cable 203 has a characteristic impedance of less than 15 Ω. Thus, reduction in the operability of the probe 1 can be suppressed by suppressing excessive increase in the outer diameter D of the inner conductor 3a. Further, reduction in the pressure resistance of the coaxial cable 203 can be suppressed by suppressing excessive reduction in the thickness t of the insulator 3b.

The other effect of the photoacoustic imaging apparatus 200 according to the second embodiment is the same as that achieved by the photoacoustic imaging apparatus 100 according to the first embodiment.

(Third Embodiment)

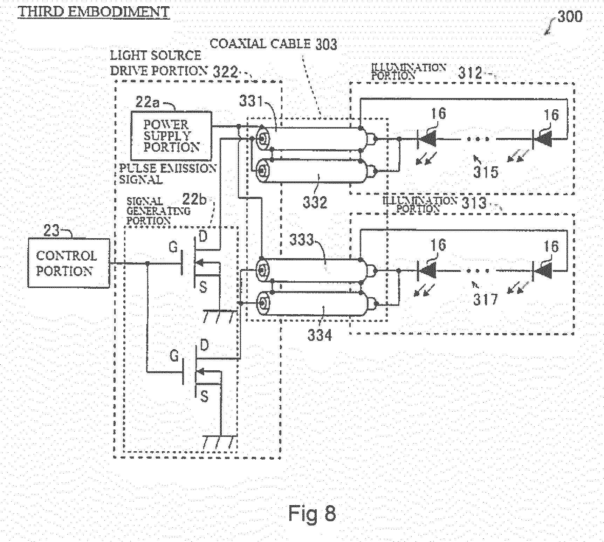

The configuration of a photoacoustic imaging apparatus 300 according to a third embodiment of the present invention will be described next by referring to FIG. 8. In the third embodiment, unlike in the photoacoustic imaging apparatus according to each of the first and second embodiments where each of two light source portions of a probe and an apparatus body are connected to each other through one coaxial cable, each of the two light source portions of the probe and the apparatus body are connected to each other through two (a plurality of) coaxial cables.

As shown in FIG. 8, the photoacoustic imaging apparatus 300 according to the third embodiment includes a coaxial cable 303. The photoacoustic imaging apparatus 300 includes an illumination portion 312 including a light source portion 315, an illumination portion 313 including a light source portion 317, and a light source drive portion 322.

The coaxial cable 303 includes a first coaxial cable 331 and a second coaxial cable 332 through which the light source portion 315 and the light source drive portion 322 are connected. The first coaxial cable 331 and the second coaxial cable 332 are connected in parallel to the light source portion 315 and the light source drive portion 322. The first coaxial cable 331 and the second coaxial cable 332 are configured in such a manner that, in response to supply of power from the light source drive portion 322 to the light source portion 315, the sum of the value of a current (peak value) flowing in the first coaxial cable 331 and that of a current (peak value) flowing in the second coaxial cable 332 is 10 A or more.

The coaxial cable 303 includes a third coaxial cable 333 and a fourth coaxial cable 334 through which the light source portion 317 and the light source drive portion 322 are connected. The third coaxial cable 333 and the fourth coaxial cable 334 are connected in parallel to the light source portion 317 and the light source drive portion 322. The third coaxial cable 333 and the fourth coaxial cable 334 are configured in such a manner that, in response to supply of power from the light source drive portion 322 to the light source portion 317, the sum of the value of a current (peak value) flowing in the third coaxial cable 333 and that of a current (peak value) flowing in the fourth coaxial cable 334 is 10 A or more.

The first coaxial cable 331, the second coaxial cable 332, the third coaxial cable 333, and the fourth coaxial cable 334 are configured equally to have a characteristic impedance of about 50 Ω. The respective characteristic impedances of the first coaxial cable 331 and the second coaxial cable 332 connected in parallel are combined, so that a characteristic impedance of 25 Ω is formed in each of the first coaxial cable 331 and the second coaxial cable 332. The respective characteristic impedances of the third coaxial cable 333 and the fourth coaxial cable 334 are also combined, so that a characteristic impedance of 25 Ω is formed in each of the third coaxial cable 333 and the fourth coaxial cable 334.

Specifically, like the coaxial cable 203 according to the second embodiment, the coaxial cable 303 has a characteristic impedance of 30 Ω or less (see FIG. 7). This can suppress reduction in the responsivity of a current flowing in the light-emitting diode element 16 to a greater degree.

The configuration of the photoacoustic imaging apparatus 300 according to the third embodiment is the same in the other respects as the photoacoustic imaging apparatus 100 according to the first embodiment.

The third embodiment achieves the following effect.

As described above, in the third embodiment, the probe 1 and the apparatus body 2 are connected through the first coaxial cable 331, the second coaxial cable 332, the third coaxial cable 333, and the fourth coaxial cable 334. A coaxial cable commonly used has a characteristic impedance of 50 Ω (or 75 Ω). As a result of the aforementioned configuration, the characteristic impedance of the coaxial cable 303 can be set easily at a value lower than 50 Ω (or 75 Ω) by using a commonly-used (general-purposed) coaxial cable, without the need for using a dedicated (customized) coaxial cable. The other effect of the photoacoustic imaging apparatus 300 according to the third embodiment is the same as that achieved by the photoacoustic imaging apparatus 100 according to the first embodiment.

(Fourth Embodiment)

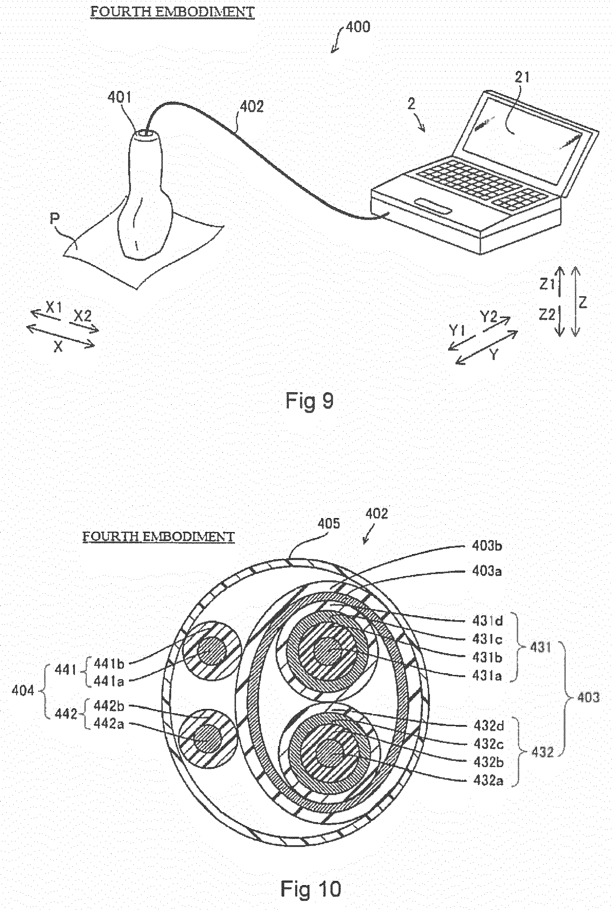

The configuration of a photoacoustic imaging apparatus 400 according to a fourth embodiment of the present invention will be described next by referring to FIGS. 9 to 11. In the fourth embodiment, unlike in the photoacoustic imaging apparatus according to each of the first to third embodiments where a signal cable and a coaxial cable are connected to a probe and an apparatus body so as to be routed separately, the signal cable and the coaxial cable are connected to the probe and the apparatus body so as to be routed integrally.

As shown in FIG. 9, the photoacoustic imaging apparatus 400 according to the fourth embodiment includes a probe 401 and a cable 402. A coaxial cable 403 and a signal cable 404 are provided inside the cable 402. The coaxial cable 403 and the signal cable 404 are configured to be routed in an integrated state.

More specifically, as shown in FIG 10, the cable 402 includes a jacket 405 provided to cover the coaxial cable 403 and the signal cable 404. The signal cable 404 includes a plurality of (128) cables connected to corresponding ones of the aforementioned channels of the ultrasonic vibrator. Each of the cables is configured to achieve transmission of a signal when the signal is transmitted and received between a corresponding one of the channels of the ultrasonic vibrator, the control portion 23, and the imaging portion 24.

To facilitate description, FIG. 10 shows only a signal cable 441 and a signal cable 442 belonging to the signal cable 404. The signal cable 441 is formed of a conductor 441 a and a jacket 441b (insulator) covering an outer peripheral surface of the conductor 441a. Like the signal cable 441, the signal cable 442 is formed of a conductor 442a and a jacket 442b (insulator) covering an outer peripheral surface of the conductor 442a.

In the fourth embodiment, the cable 402 includes a shield 403a covering the outside of the coaxial cable 403. The shield 403a is an example of a "first shield" according to the present invention.

More specifically, the coaxial cable 403 includes a coaxial cable 431 and a coaxial cable 432. The coaxial cable 431 includes an inner conductor 431 a, an insulator 431b, an outer conductor 431c, and a jacket 431d (insulator) arranged in this order in an inside-to-outside fashion. Like the coaxial cable 431, the coaxial cable 432 includes an inner conductor 432a, an insulator 432b, an outer conductor 432c, and a jacket 432d (insulator).

The shield 403a is made of metal and is configured to function as a shield against an electromagnetic wave. The shield 403a is provided to integrally cover the outside of the coaxial cable 431 and the outside of the coaxial cable 432 that are arranged adjacent to each other. The shield 403a may be grounded.

The cable 402 includes a jacket 403b arranged inside the jacket 405 and covering an outer peripheral surface of the shield 403a.

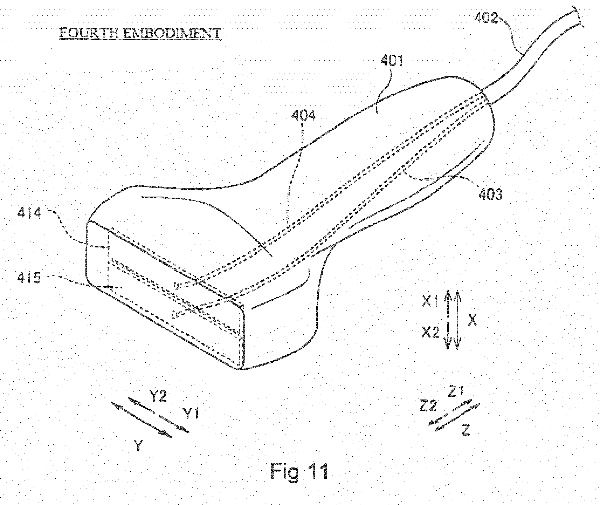

As shown in FIG. 11, the probe 401 is configured in such a manner that a light source portion 415 and an acoustic wave detecting portion 414 are arranged inside the probe 401. The coaxial cable 403 is connected to the light source portion 415. The signal cable 404 is connected to the acoustic wave detecting portion 414.

The configuration of the photoacoustic imaging apparatus 400 according to the fourth embodiment is the same in the other respects as the photoacoustic imaging apparatus 100 according to the first embodiment.

The fourth embodiment achieves the following effect.

As described above, in the fourth embodiment, the coaxial cable 403 and the signal cable 404 are configured to be routed in an integrated state. This prevents separation between the coaxial cable 403 and the signal cable 404. Thus, the operability of the probe 1 can be increased, compared to a configuration where the coaxial cable 403 and the signal cable 404 are routed separately.

As described above, in the fourth embodiment, the cable 402 includes the shield 403a covering the outside of the coaxial cable 403. This allows the shield 403a to function as a shield against an electromagnetic wave. This makes it possible to shield an electromagnetic wave (noise) to enter the coaxial cable 403 covered by the shield 403a and an electromagnetic wave to be emitted from the coaxial cable covered by the shield 403a.

The other effect of the photoacoustic imaging apparatus 400 according to the fourth embodiment is the same as that achieved by the photoacoustic imaging apparatus 100 according to the first embodiment.

(Fifth Embodiment)

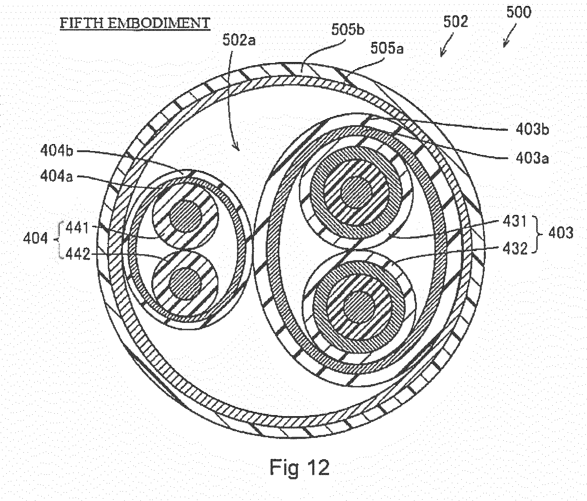

The configuration of a photoacoustic imaging apparatus 500 according to a fifth embodiment of the present invention will be described next by referring to FIG. 12. In the fifth embodiment, a cable 502 includes a shield 505a covering the outside of a cable group 502a formed of a coaxial cable 403 and a signal cable 404.

As shown in FIG. 12, the photoacoustic imaging apparatus 500 according to the fifth embodiment includes the cable 502. The cable 502 includes the coaxial cable 403 and the signal cable 404.

In the fifth embodiment, the cable 502 includes a shield 403a covering the outside of the coaxial cable 402, and a shield 404a covering the outside of the signal cable 402. The shields 403a and 404a are examples of the "first shield" according to the present invention.

The shield 403a is configured equally to the shield 403a of the photoacoustic imaging apparatus 400 according to the fourth embodiment. Like the shield 403a, the shield 404a is made of metal and is configured to function as a shield against an electromagnetic wave. The signal cable 404 includes a signal cable 441 and a signal cable 442. The shield 404a is arranged to cover the signal cables 441 and 442 from outside arranged adjacent to each other. The cable 502 includes a jacket 403b (insulator) covering an outer peripheral surface of the shield 403a, and a jacket 404b covering an outer peripheral surface of the shield 404a.

In the fifth embodiment, the coaxial cable 403 and the signal cable 404 form the cable group 502a routed in an integrated state. The cable 502 includes the shield 505a covering the outside of the cable group 502a. The shield 505a is an example of a "second shield" according to the present invention.

More specifically, the cable group 502a is formed of the coaxial cable 403, the shield 403a, the jacket 403b, the signal cable 404, the shield 404a, and the jacket 404b. The cable 502 includes the shield 505a covering the cable group 502a so as to surround the outside of the cable group 502a. The shield 505 is made of metal and is configured to function as a shield against an electromagnetic wave. The cable 502 includes the jacket 505b covering an outer peripheral surface of the shield 505a. In this way, the cable group 505a is configured to be routed as an integrated group by the presence of the jacket 505b while being shielded from an electromagnetic wave by the shield 505a.

The configuration of the photoacoustic imaging apparatus 500 according to the fifth embodiment is the same in the other respects as the photoacoustic imaging apparatus 100 according to the first embodiment.

The fifth embodiment achieves the following effect.

As described above, in the fifth embodiment, the coaxial cable 403 and the signal cable 404 are configured to form the cable group 502a routed in an integrated state. Further, the cable 502 includes the shield 505a covering the outside of the cable group 502a. This allows the shield 505a to function as a shield against an electromagnetic wave. This makes it possible to shield an electromagnetic wave (noise) to enter the cable group 502a from outside and an electromagnetic wave to be emitted to the outside of the cable group 502a.

The other effect of the photoacoustic imaging apparatus 500 according to the fifth embodiment is the same as that achieved by the photoacoustic imaging apparatus 100 according to the first embodiment.

(Sixth Embodiment)

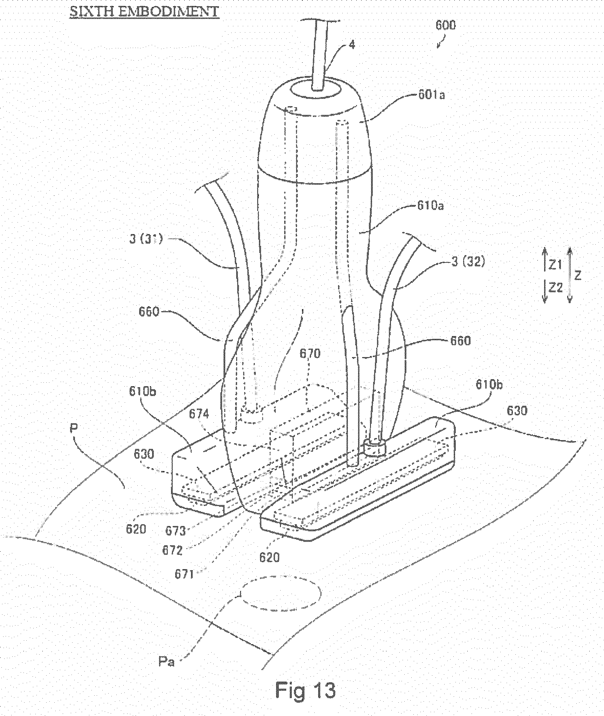

The configuration of a photoacoustic imaging apparatus 600 according to a sixth embodiment of the present invention will be described next by referring to FIGS. 13 to 18.

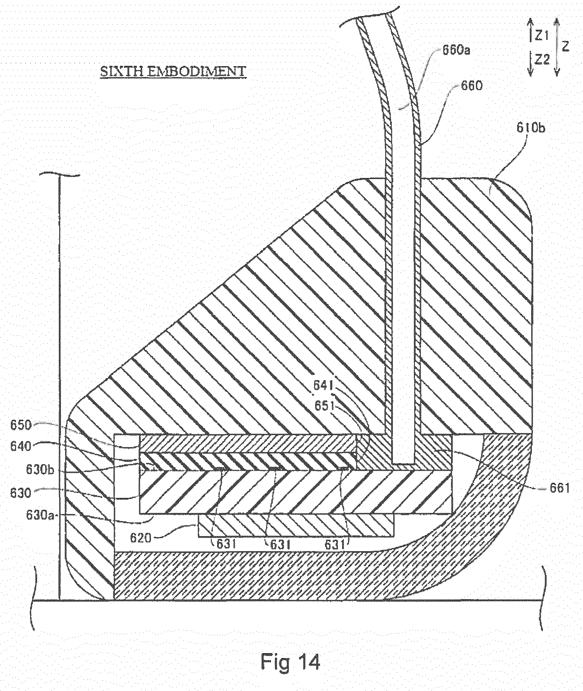

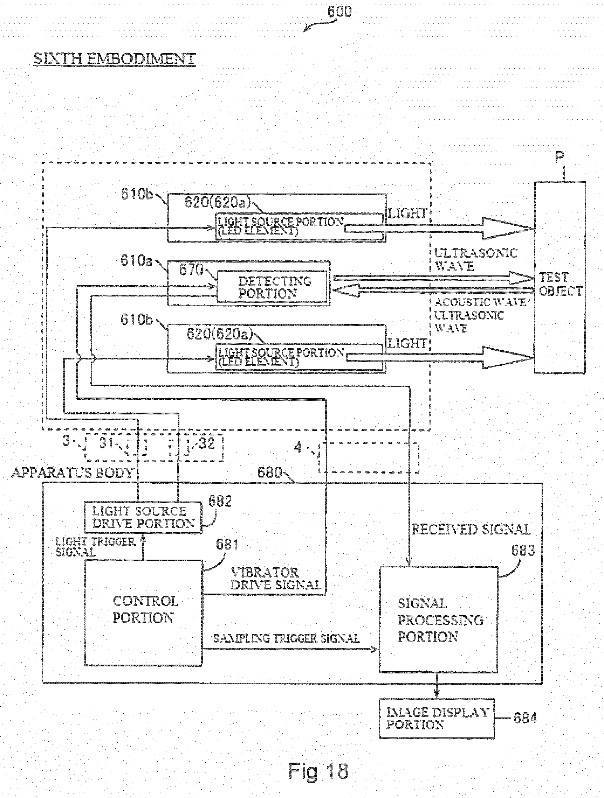

As shown in FIG. 13, the photoacoustic imaging apparatus 600 according to the sixth embodiment of the present invention includes a first housing 610a, a second housing 610b, a light source portion 620, and a substrate 630. As shown in FIG. 14, the photoacoustic imaging apparatus 600 includes an insulating member 640, an electromagnetic wave absorption layer 650, a heat conducting portion 660, a detecting portion 670 (see FIG. 13), and an apparatus body 680 (see FIG. 18). For the convenience of explanation, the insulating member 640 and the electromagnetic wave absorption layer 650 are omitted from FIG. 13.

The first housing 610a and the second housing 610b are made of resin. The first housing 610a is an example of a "housing" according to the present invention.

As shown in FIG 13, the first housing 610a houses the detecting portion 670. The first housing 610a includes a heat dissipating portion 601 a formed at an upper part (on a Z1 side) of the first housing 610a. The heat dissipating portion 601a is made of metal such as aluminum, for example. A shape applicable as the first housing 610a (photoacoustic imaging apparatus 600) includes a streamline shape, a convex shape, and a sector shape, for example.

The second housing 610b houses the light source portion 620, the substrate 630, the insulating member 640 (see FIG. 14), and the electromagnetic wave absorption layer 650 (see FIG. 14). The second housing 610b includes a pair of second housings 610b arranged so as to sandwich the first housing 610a therebetween. A part of the second housing 610b on a Z2 side is configured to allow transmission of light.

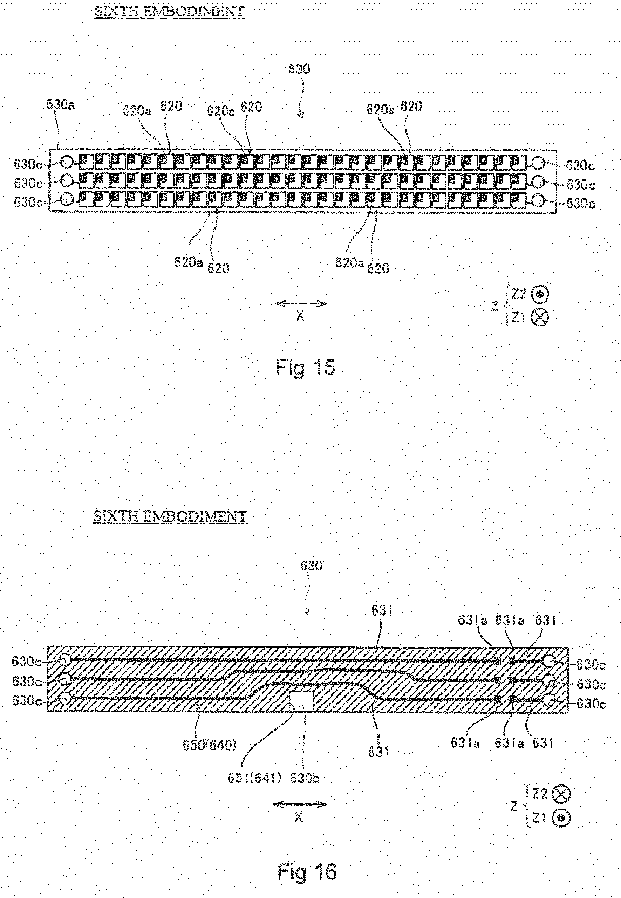

The light source portion 620 is arranged near the detecting portion 670. As shown in FIG 15, the light source portion 620 is provided closer to a first surface 630a of the substrate 630 (on a Z2 side). The light source portion 620 includes a plurality of light-emitting elements 620a. The light-emitting elements 620a are each formed of an LED element (light-emitting diode element). The light-emitting elements 620a adjacent to each other in the lengthwise direction (direction X) of the substrate 630 are connected to each other through a bonding wire (not shown in the drawings). All the light-emitting elements 620a are connected in series. The light source portion 620 is configured to emit light to a test object P (see FIG. 13). A detecting target Pa in the test object P (see FIG. 13) generates an acoustic wave when the detecting target Pa absorbs light emitted from the light source portion 620. The first surface 630a of the substrate 630 is a concept indicating a surface of the substrate 630 facing the test object P in a state of use shown in FIG. 13.

An ultrasonic wave referred to in this description denotes a sound wave (elastic wave) having such a high frequency as not to cause a sensation of hearing of a person with normal hearing ability and is a concept of a sound wave of about 16000 Hz or more. In this description, light emitted from the light source portion 620 is absorbed by the detection target Pa in the test object P to generate an ultrasonic wave and this ultrasonic wave will be called an "acoustic wave." An ultrasonic wave generated by the detecting portion 670 (an ultrasonic vibrator 673 described later) and reflected on the detection target Pa in the test object P will simply be called an "ultrasonic wave."

The substrate 630 is a plate-like aluminum substrate. The substrate 630 has a surface covered with a coating film made of an insulator. The substrate 630 has a rectangular shape extending in the direction X in a plan view. The substrate 630 is configured in such a manner that the light source portion 620 is arranged on the first surface 630a. The substrate 630 is housed in the second housing 610b in such a manner that the first surface 630a faces the test object P. In the state of use shown in FIG. 13, the substrate 630 is arranged below the ultrasonic vibrator 673 (see FIG. 13) of the detecting portion 670 described later. As shown in FIG. 16, a second surface 630b of the substrate 630 on the opposite side (Z1 side) to the first surface 630a is provided with a wire 631. The wire 631 is provided on the coating film made of the insulator. Unlike arranging both the light source portion 620 and the wire 631 on the first surface 630a, arranging the light source portion 620 on the first surface 630a and arranging the wire 631 on the second surface 630b makes it possible to reduce the size (area) of the substrate 630 in a plan view. As a result, the second housing 610b can be formed into a compact size.

The wire 631 may be formed of a wire made of metal such as copper, for example. Alternatively, the wire 631 may be a wiring pattern formed on the second surface 630b. The light source portion 620 is electrically connected to the wire 631 through through holes 630c at opposite ends of the light source portion 620 in the direction X. The wire 631 is connected to the coaxial cable 3 (31 and 32) at a connecting portion 631a (see FIG. 13). The coaxial cable 3 is treated so as not to generate an electromagnetic wave.

As shown in FIG. 14, the insulating member 640 is formed of a film member. The insulating member 640 is formed by using a material made of an insulator. For example, a polyimide film is applicable as the insulating member 640. The insulating member 640 has a rectangular shape having a length extending in the direction X in a plan view (see FIG. 16). The insulating member 640 is provided between the second surface 630b of the substrate 630 and the electromagnetic wave absorption layer 650. The insulating member 640 is provided to cover the second surface 630b of the substrate 630 substantially entirely (see FIG. 16). The insulating member 640 is tightly attached to each of the second surface 630b of the substrate 630 and the electromagnetic wave absorption layer 650. The insulating member 640 is provided to cover the wire 631 of the substrate 630. The insulating member 640 is arranged to be tightly attached to the wire 631. The insulating member 640 is bonded with an adhesive to each of the second surface 630b of the substrate 630 and the electromagnetic wave absorption layer 650. The insulating member 640 is provided with a substrate exposing portion 641 like a cutout formed for partially exposing the second surface 630b of the substrate 630.

The electromagnetic wave absorption layer 650 is formed of a sheet-like member. The electromagnetic wave absorption layer 650 is configured to cover the wire 631 from a place adjacent to the second surface 630b of the substrate 630 (from the Z1 side). The electromagnetic wave absorption layer 650 is configured to cover a surface of the insulating member 640 (on the Z1 side) substantially entirely that is opposite a surface thereof closer to the substrate 630 (see FIG. 16). The electromagnetic wave absorption layer 650 is bonded through the insulating member 640 to the second surface 630b of the substrate 630. The electromagnetic wave absorption layer 650 is provided to cover the wire 631 and the second surface 630b of the substrate 630 substantially entirely with the intervention of the insulating member 640. The electromagnetic wave absorption layer 650 is provided with a substrate exposing portion 651 like a cutout (see FIG. 16) formed for partially exposing the second surface 630b of the substrate 630. The substrate exposing portion 651 is provided at a position corresponding to the substrate exposing portion 641 of the insulating member 640.

The electromagnetic wave absorption layer 650 contains a magnetic substance and a dielectric substance. Ferromagnetic metal, ferromagnetic alloy, a ferromagnetic sintered body, and ferromagnetic oxide are applicable as the magnetic body, for example. More specifically, Fe, Ni, Co, and Gd are applicable as the ferromagnetic metal, for example. Permalloy and supermalloy as Fe-Ni alloys, permendur (Fe-Co alloy), sendust (Fe-Si-Al alloy), SmCo, and NdFeB are applicable as the ferromagnetic alloy, for example. A sintered body of a ferromagnetic substance is also applicable. Various ferrite-based materials are applicable as the ferromagnetic oxide. The magnetic substance absorbs, particularly a magnetic-field component in an electromagnetic wave, and converting the absorbed component to heat. Rubber, resin, glass, and ceramic are applicable as the dielectric substance, for example. The dielectric substance absorbs, particularly an electric-field component in an electromagnetic wave, and converting the absorbed component to heat.

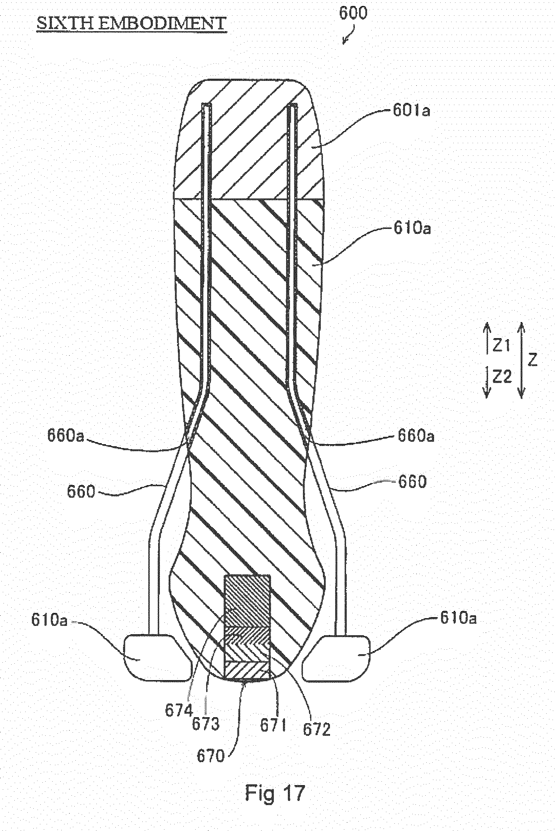

The heat conducting portion 660 is formed of a heat pipe having an inner cavity 660a. The heat conducting portion 660 is made of metal such as copper, for example. The heat conducting portion 660 has a large-diameter portion 661 formed at one end thereof (on the Z2 side). This allows increase in the area of contact with the substrate 630, so that heat can be drawn efficiently from the substrate 630. The heat conducting portion 660 directly contacts the second surface 630b of the substrate 630 at the one end (large-diameter portion 661) with the intervention of the substrate exposing portion 651 of the electromagnetic wave absorption layer 650 and the substrate exposing portion 641 of the insulating member 640, thereby drawing heat from the substrate 630. A surface of the heat conducting portion 660 at the one end (large-diameter portion 661) is insulating treated. As shown in FIG 17, the heat conducting portion 660 contacts the first housing 610a at an opposite end (on the Z1 side). More specifically, the opposite end (on the Z1 side) of the heat conducting portion 660 is connected to the heat dissipating portion 601a of the first housing 610a. The heat conducting portion 660 is configured to move heat from a high-temperature side (Z2 side) contacting the substrate 630 toward a low-temperature side (Z1 side).

The detecting portion 670 is configured to detect an acoustic wave generated from the detection target Pa in the test object P (see FIG. 13) when the detection target Pa absorbs light emitted from the light source portion 620. The detecting portion 670 includes an acoustic lens 671, an acoustic matching layer 672, the ultrasonic vibrator 673, and a backing material 674. The detecting portion 670 is configured to emit an ultrasonic wave. The detecting portion 670 is configured to detect an ultrasonic wave and an acoustic wave.

The detecting portion 670 detects an acoustic wave (ultrasonic wave) generated from the detection target Pa in the test object P when light is emitted from the light source portion 620 to the test object P. The photoacoustic imaging apparatus 600 is configured to be capable of forming an image of the detection target Pa based on the acoustic wave detected by the detecting portion 670.

The detecting portion 670 is configured to emit an ultrasonic wave from the ultrasonic vibrator 673 to the test object P. The detecting portion 670 is further configured to be capable of detecting the ultrasonic wave reflected on the detection target Pa in the test object P. The photoacoustic imaging apparatus 600 is configured to be capable of forming an image of the detection target based on the reflected ultrasonic wave detected by the detecting portion 670.

The acoustic lens 671 (see FIG. 13) is configured to apply an ultrasonic wave from the acoustic matching layer 672 (ultrasonic vibrator 673) to the test object P while focusing the ultrasonic wave.

The acoustic matching layer 672 (see FIG. 13) is formed of a plurality of layers having different acoustic impedances. The acoustic matching layer 672 is configured to match the acoustic impedance of the ultrasonic vibrator 673 and the acoustic impedance of the test object P.

The ultrasonic vibrator 673 (see FIG. 13) is formed of a piezoelectric element (made of lead zirconate titanate (PZT), for example), etc. The ultrasonic vibrator 673 vibrates in response to application of a voltage to generate an ultrasonic wave. If an acoustic wave (ultrasonic wave) is detected, the ultrasonic vibrator 673 vibrates to generate a voltage (received signal). The ultrasonic vibrator 673 also generates a voltage in response to an electromagnetic wave generated from the light source portion 620 and the wire 631, and vibrates to generate an ultrasonic wave. This ultrasonic wave resulting from the electromagnetic wave is reflected on the detection target Pa in the test object P and the reflected ultrasonic wave is detected by the ultrasonic vibrator 673 (detecting portion 670), thereby causing inclusion of noise in an image to be formed.

The backing material 674 (see FIG. 13) is arranged behind the ultrasonic vibrator 673 (on the Z1 side). The backing material 674 is configured to suppress backward propagation of an ultrasonic wave and that of an acoustic wave.

As shown in FIG. 18, the apparatus body 680 includes a control portion 681, a light source drive portion 682, a signal processing portion 683, and an image display portion 684.

The control portion 681 includes a CPU (central processing unit), etc., and is configured to operate according to a certain program. The control portion 681 is configured to be responsible for overall control of the photoacoustic imaging apparatus 600.

The light source drive portion 682 is configured to acquire power from external power supply portion (not shown in the drawings). The light source drive portion 682 is arranged in the apparatus body 680 separate from the first housing 610a housing the detecting portion 670. This allows the detecting portion 670 to be less likely to detect noise due to an electromagnetic wave emitted from the light source drive portion 682. The light source drive portion 682 is configured to acquire a light trigger signal from the control portion 681 and supply power to the light source portion 620 based on the acquired light trigger signal. The light source drive portion 682 is configured to supply the power based on the light trigger signal to the light source portion 620 through the coaxial cable 3 (see FIG. 13) connected to the wire 631 of the substrate 630. The light source drive portion 682 is configured to make the light source portion 620 generate pulsed light of a pulse width of about 150 ns, for example.

The signal processing portion 683 is configured to acquire a sampling trigger signal synchronized with a light trigger signal from the control portion 681. The signal processing portion 683 is configured to acquire a voltage (received signal) from the detecting portion 670 generated when the ultrasonic vibrator 673 detects an acoustic wave (ultrasonic wave) to vibrate. The signal processing portion 683 is configured to form a tomographic image responsive to the acoustic wave and a tomographic image responsive to the ultrasonic wave based on the acquired sampling trigger signal and the acquired received signal, to execute processing of combining the tomographic images, and to output the composite image to the image display portion 684.

The image display portion 684 is formed of a liquid crystal panel, etc. The image display portion 684 is configured to display the composite image.

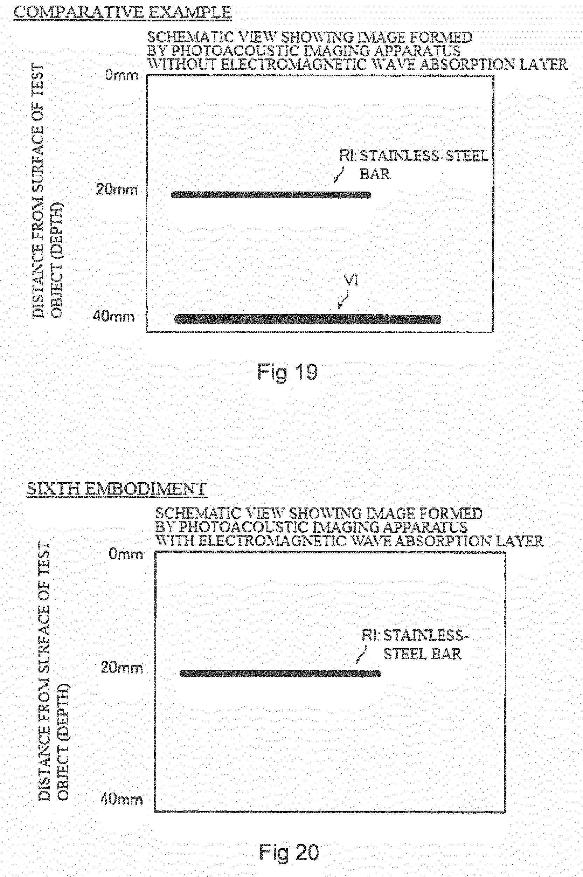

Described next by referring to FIGS. 19 and 20 is a result of experiment conducted to confirm effect achieved by providing the electromagnetic wave absorption layer 650 according to the sixth embodiment. This experiment was conducted by using a stainless-steel bar as the detection target Pa and forming the test object P by burying the stainless-steel bar into agar to a depth position of 20 mm from a surface of the agar.

A speed Vc of light ($3 \times 10^8$ m/s) emitted from the light source portion 620 is extremely higher than a speed Vs of an acoustic wave (ultrasonic wave) (Vc >> Vs). Thus, time t1 from emission of light from the light source portion 620 to arrival of the light at the detection target Pa is extremely shorter than time t2 for an ultrasonic wave generated from the detecting portion 670 to arrive at the detection target Pa and time t3 for an acoustic wave (ultrasonic wave) to arrive at the detecting portion 670 from the ultrasonic wave detection target Pa (t3≈t2). Thus, t1 can be considered to be substantially zero in terms of relationships with t2 and t3.

FIG. 19 schematically shows images formed by a photoacoustic imaging apparatus without an electromagnetic wave absorption layer. In the case of using the photoacoustic imaging apparatus without an electromagnetic wave absorption layer, a real image R1 indicating the stainless-steel bar (detection target Pa) was observed at a position of 20 mm from the surface of the agar. If light is emitted from the light source portion 620 to the stainless-steel bar, an acoustic wave from the stainless-steel bar is detected after elapse of t1 + t3 (≈t3) from a point in time t0 of emission of the light. The real image R1 is an image resulting from this acoustic wave.

In the case of using the photoacoustic imaging apparatus without an electromagnetic wave absorption layer, a virtual image V1 was observed at a position of 40 mm from the surface of the agar (a position where the stainless-steel bar does not exist). If light is emitted from the light source portion to the stainless-steel bar, an electromagnetic wave is generated from the light source portion and the wire simultaneously with the point in time t0 of emission of the light. Specifically, the ultrasonic wave vibrator of the detecting portion vibrates at the point in time t0. In this way, an ultrasonic wave reflected on the stainless-steel bar is detected after elapse of t2 + t3 (≈2×t3) from the point in time t0. The virtual image V1 is formed at a position twice as deep as the depth position of the real image R1 (a position from the surface of the test object P). The virtual image V1 is an image resulting from this ultrasonic wave. The virtual image V1 is noise due to the electromagnetic wave generated from the light source portion and the wire simultaneously with emission of the light.

FIG. 20 schematically shows an image formed by the photoacoustic imaging apparatus 600 with the electromagnetic wave absorption layer 650. In the case of using the photoacoustic imaging apparatus 600 with the electromagnetic wave absorption layer 650, only the real image R1 indicating the stainless-steel bar (detection target Pa) was observed at a position of 20 mm from the surface of the agar. An acoustic wave from the stainless-steel bar is detected after elapse of t1 + t3 (≈t3) from the point in time t0 of emission of light. The real image R1 is an image resulting from this acoustic wave.

Unlike in the case of using the photoacoustic imaging apparatus without an electromagnetic wave absorption layer (FIG. 19), the virtual image V1 was not observed at a position of 40 mm from the surface of the agar (a position where the stainless-steel bar does not exist) in the case of using the photoacoustic imaging apparatus 600 with the electromagnetic wave absorption layer 650. This confirms that the electromagnetic wave absorption layer 650 absorbed an electromagnetic wave generated from the light source portion 620 and the wire 631 simultaneously with the point in time t0 of emission of light from the light source portion 620.

The sixth embodiment achieves the following effect.

As described above, in the sixth embodiment, the electromagnetic wave absorption layer 650 is provided to cover the wire 631 from a place adjacent to the second surface 630b of the substrate 630. It is probable that the likelihood of entry of an electromagnetic wave, etc. (noise) from outside will not be reduced sufficiently and inside-to-outside emission of an electromagnetic wave will not be suppressed sufficiently for a structure other than the coaxial cable 3, specifically, the light source portion 620 including the light-emitting element 620a. In this regard, in the sixth embodiment, the photoacoustic imaging apparatus 600 includes the electromagnetic wave absorption layer 650 covering the wire 631 from a place adjacent to the second surface 630b of the substrate 630. This allows the electromagnetic wave absorption layer 650 to absorb an electromagnetic wave generated from the light source portion 620 and the wire 631 connected to the light source portion 620 to travel toward the detecting portion 670 near the light source portion 620. Thus, the detecting portion 670 is allowed to be less likely to detect an electromagnetic wave, making it possible to reduce the likelihood of inclusion of noise in an image to be formed by the photoacoustic imaging apparatus 600.

In the sixth embodiment, the electromagnetic wave absorption layer 650 has the substrate exposing portion 651 for exposing the second surface 630b of the substrate 630. Further, the heat conducting portion 660 for dissipating heat from the substrate 630 is arranged to contact the second surface 630b of the substrate 630 through the substrate exposing portion 651 of the electromagnetic wave absorption layer 650. Thus, heat generated from the light source portion 620 can be dissipated effectively by the heat conducting portion 660 through the second surface 630b of the substrate 630. As a result, the lifetime of the light source portion 620 can be extended.

In the sixth embodiment, the first housing 610a housing the detecting portion 670 is provided. The heat conducting portion 660 is configured to contact the second surface 630b of the substrate 630 at the one end thereof and to contact the first housing 610a at the opposite end thereof. This allows the heat conducting portion 660 to transfer heat generated from the light source portion 620 toward the first housing 610a. As a result, the heat generated from the light source portion 620 can be dissipated more effectively.

In the sixth embodiment, the first housing 610a includes the heat dissipating portion 601a. The heat conducting portion 660 is connected at its opposite end to the heat dissipating portion 601 a. This allows the heat dissipating portion 601a adjacent to the opposite end of the heat conducting portion 660 to still more effectively dissipate heat generated from the light source portion 620.

In the sixth embodiment, the insulating member 640 is provided between the second surface 630b of the substrate 630 and the electromagnetic wave absorption layer 650. This allows the insulating layer to function to increase an insulation breakdown voltage. As a result, a high voltage can be applied to the light source portion 620, so that the intensity of light emitted from the light source portion 620 can be increased.

In the sixth embodiment, the light-emitting element 620a of the light source portion 620 is formed of a light-emitting diode element. This allows reduction in the power consumption of the light source portion 620 and size reduction of the apparatus, compared to forming the light source portion 620 using a solid-state laser source.

(Seventh Embodiment)

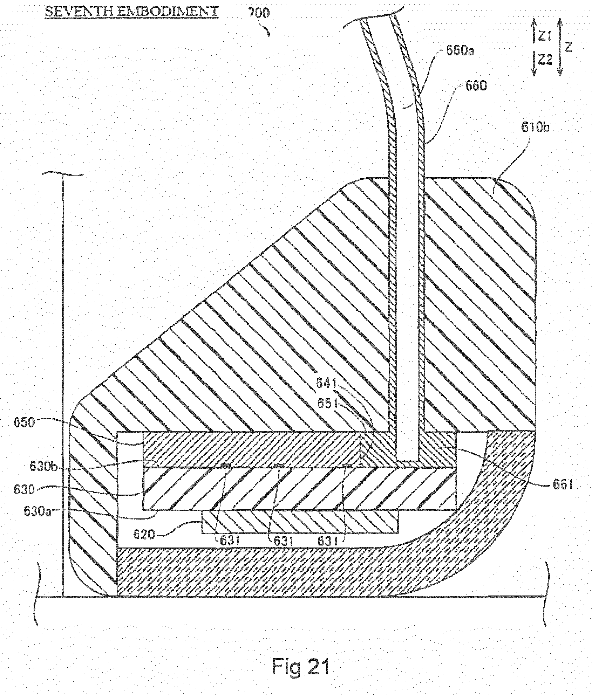

The configuration of a photoacoustic imaging apparatus 700 according to a seventh embodiment of the present invention will be described next by referring to FIG. 21.

In the photoacoustic imaging apparatus 700 according to the seventh embodiment described herein, unlike in the sixth embodiment where the insulating member 640 is provided between the second surface 630b of the substrate 630 and the electromagnetic wave absorption layer 650, the insulating member 640 is not provided between the second surface 630b of the substrate 630 and the electromagnetic wave absorption layer 650. In the description of the seventh embodiment, structures comparable to those of the sixth embodiment will be given the same signs and description of such structures will be omitted.

As shown in FIG 21, in the photoacoustic imaging apparatus 700 according to the seventh embodiment, the electromagnetic wave absorption layer 650 is provided to contact the second surface 630b of the substrate 630. The electromagnetic wave absorption layer 650 is configured to cover the second surface 630b of the substrate 630 substantially entirely. A surface of the electromagnetic wave absorption layer 650 on a Z2 side is insulating treated. The electromagnetic wave absorption layer 650 is provided to directly cover the wire 631 and the second surface 630b of the substrate 630. The electromagnetic wave absorption layer 650 is arranged to be tightly attached to (to contact) the wire 631 and the second surface 630b of the substrate 630. The electromagnetic wave absorption layer 650 is bonded with an adhesive to the second surface 630b of the substrate 630.

In the seventh embodiment, the electromagnetic wave absorption layer 650 is allowed to be tightly attached to (to contact) the wire 631 and the second surface 630b of the substrate 630. Thus, an electromagnetic wave emitted from the wire 631 can be absorbed reliably. In the seventh embodiment, unlike the configuration of providing the insulating member 640, the configuration of the photoacoustic imaging apparatus 700 can be simplified. Unlike the configuration of providing the insulating member 640, a parts count can be reduced. In the sixth embodiment, by the provision of the sheet-like insulating member 640, a higher voltage can be applied easily to the light source portion 620 than in the seventh embodiment. In this way, the intensity of light emitted from the light source portion 620 can be increased easily.

The configuration according to the seventh embodiment is the same in the other respects as the aforementioned configuration according to the sixth embodiment.

The seventh embodiment achieves the following effect.

Like in the sixth embodiment, in the seventh embodiment, the detecting portion 670 is allowed to be less likely to detect an electromagnetic wave, making it possible to reduce the likelihood of inclusion of noise in an image to be formed by the photoacoustic imaging apparatus 700.

(Eighth Embodiment)

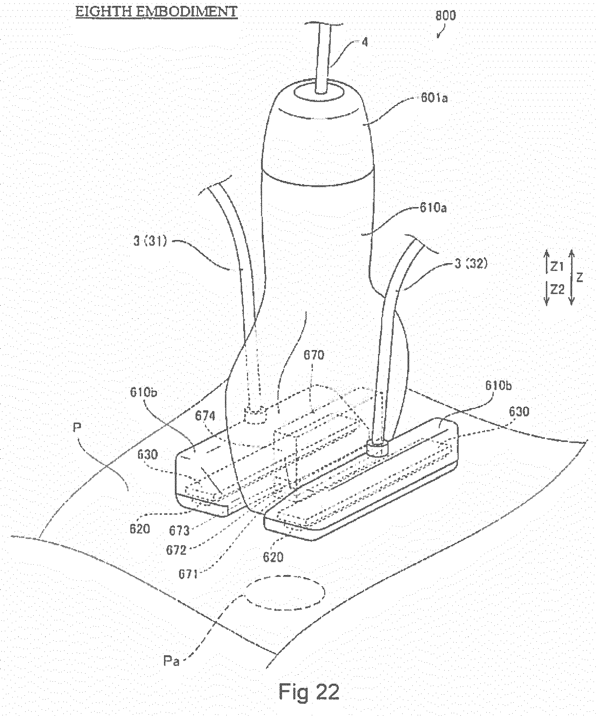

The configuration of a photoacoustic imaging apparatus 800 according to an eighth embodiment of the present invention will be described next by referring to FIG. 22.

In the photoacoustic imaging apparatus 800 according to the eighth embodiment described herein, unlike in the sixth embodiment with the heat conducting portion 660, the heat conducting portion 660 is not provided. In the description of the eighth embodiment, structures comparable to those of the sixth embodiment will be given the same signs and description of such structures will be omitted.

As shown in FIG. 22, the photoacoustic imaging apparatus 800 according to the eighth embodiment includes a first housing 610a, a second housing 610b, a light source portion 620, a substrate 630. The photoacoustic imaging apparatus 800 includes an insulating member 640 (see FIG. 14), an electromagnetic wave absorption layer 650 (see FIG. 14), a detecting portion 670, and an apparatus body 680 (see FIG. 18).

In the eighth embodiment, unlike the configuration of providing the heat conducting portion 660, the configuration of the photoacoustic imaging apparatus 800 can be simplified. Unlike the configuration of providing the heat conducting portion 660, a parts count can be reduced.

The configuration according to the eighth embodiment is the same in the other respects as the aforementioned configuration according to the sixth embodiment.

The eighth embodiment achieves the following effect.

Like in the sixth embodiment, in the eighth embodiment, the detecting portion 670 is allowed to be less likely to detect an electromagnetic wave, making it possible to reduce the likelihood of inclusion of noise in an image to be formed by the photoacoustic imaging apparatus 800.

The embodiments disclosed herein must be considered to be illustrative in all aspects and not restrictive. The range of the present invention is understood not by the above description of the embodiments but by the scope of claims for patent. All modifications within the meaning and range equivalent to the scope of claims for patent are to be embraced.

For example, in the above-described first to eighth embodiments, a light-emitting diode element is used as the light-emitting element according to the present invention. However, this is not to limit the present invention. A light-emitting element other than a light-emitting diode element can be used as the light-emitting element according to the present invention. A semiconductor laser element can be used as the light-emitting element, for example.

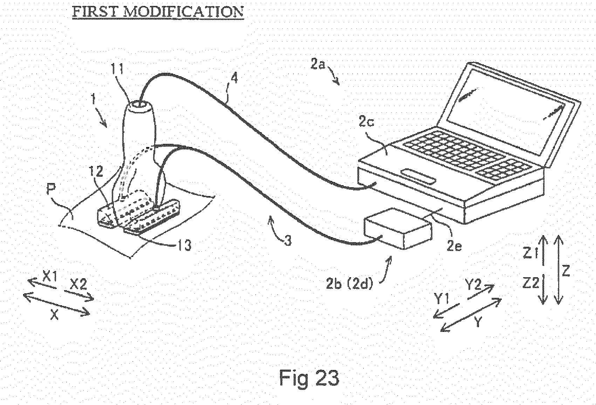

In the exemplary configuration shown in the above-described first to eighth embodiments, the light source drive portion and the imaging portion according to the present invention are provided integrally. However, this is not to limit the present invention. In the present invention, the light source drive portion and the imaging portion may be configured to be provided separately. As in a first modification shown in FIG. 23, for example, an apparatus body 2a may be configured to be formed of a light source drive portion body 2b and an imaging portion body 2c.

The apparatus body 2a according to the first modification includes the light source drive portion body 2b and the imaging portion body 2c. A light source drive portion 2d is provided inside the light source drive portion body 2b. The structures of the apparatus body 2 according to the first embodiment except the light source drive portion 2d (22) are provided inside the imaging portion body 2c.

The light source drive portion body 2b and the imaging portion body 2c are connected through a control cable 2e and are configured to transmit a light trigger signal from the imaging portion body 2c to the light source drive portion body 2b. The coaxial cable 3 is connected to the light source drive portion body 2b. The signal cable 4 is connected to the imaging portion body 2c.

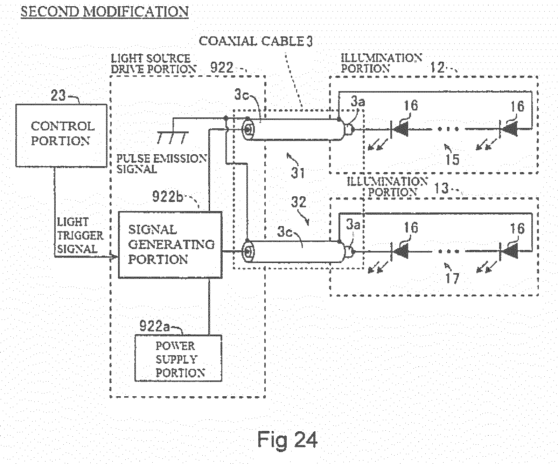

In the exemplary configuration shown in the above-described first to eighth embodiments, the outer conductor of the coaxial cable according to the present invention is connected to the power supply portion of the light source drive portion, and the inner conductor of the coaxial cable is connected to the signal generating portion. However, this is not to limit the present invention. In the present invention, the outer conductor of the coaxial cable may be configured to be grounded and the inner conductor of the coaxial cable may be configured to be connected to the signal generating portion. As in a second modification shown in FIG. 24, for example, the outer conductor 3c of the coaxial cable 3 may be configured to be grounded and the inner conductor 3a of the coaxial cable 3 may be configured to be connected to a signal generating portion 922b.

A light source drive portion 922 according to the second modification includes a power supply portion 922a and the signal generating portion 922b. The power supply portion 922a is connected to the signal generating portion 922b and is configured to be capable of applying a negative voltage (-200 V, for example). The outer conductor 3c of the coaxial cable 3 is grounded. The inner conductor 3a of the coaxial cable 3 is connected to the signal generating portion 922b.

In this configuration, if the control portion 23 places a light trigger signal at a voltage level H, the cathode of the light-emitting diode element 16 connected to the inner conductor 3a of the coaxial cable 3 is placed at a negative voltage to generate a flow of a current from the anode toward the cathode of the light-emitting diode element 16. The outer conductor 3c of the coaxial cable 3 is grounded. This reduces a potential difference from a place outside the jacket 3d (such as the ground, for example), compared to the configuration where the outer conductor 3c is connected to the power supply portion 922a (or 22a). This makes it possible to suppress increase in the thickness of the jacket 3d of the coaxial cable 3.

In the exemplary configuration shown in the above-described first to eighth embodiments, the coaxial cable according to the present invention includes one or two coaxial cables prepared for one light source portion. However, this is not to limit the present invention. In the present invention, the coaxial cable can be configured to include three or more coaxial cables.

In the exemplary configuration shown in the above-described third embodiment, the coaxial cable according to the present invention to be used includes a plurality of coaxial cables each having a characteristic impedance of 50 Ω. However, this is not to limit the present invention. In the present invention, the coaxial cable to be used can be configured to include a plurality of coaxial cables each having a characteristic impedance other than 50 Ω. For example, the coaxial cable to be used can be configured to include a plurality of coaxial cables each having a characteristic impedance of 75 Ω.

In the exemplary configuration shown in the above-described first to eighth embodiments, one or two light source portions according to the present invention are prepared for one probe. However, this is not to limit the present invention. A configuration of the present invention can be such that three or more light source portions are prepared for a probe. For example, this configuration can be such that three light source portions are prepared for a probe and the coaxial cable is connected to each of these light source portions.

The first to eighth embodiments have been described by giving exemplary numerical values according to the present invention such as a pressure resistance (250 V), the duration of a pulse of a light trigger signal (150 ns), and the length of a cable (2 m) for example. However, this is not to limit the present invention. For example, a pressure resistance may be set at 300 V, the duration of the pulse at 100 ns, and the cable at 3 m.

In the exemplary configuration shown in the above-described first to eighth embodiments, the probe according to the present invention has a streamline shape. However, this is not to limit the present invention. In the present invention, the probe can be configured to have a shape other than a streamline shape. For example, the probe may be configured to have a convex shape or a sector shape.

In the example shown in the above-described first to eighth embodiments, the probe according to the present invention includes both the light-emitting diode element (illumination portion) and the acoustic wave detecting portion (probe body). However, this is not to limit the present invention. In the present invention, the probe is not necessarily required to include both the light-emitting diode element and the acoustic wave detecting portion. For example, the probe may be configured to include the acoustic wave detecting portion and the illumination portion with the light-emitting diode element may be configured to be separated from the probe.

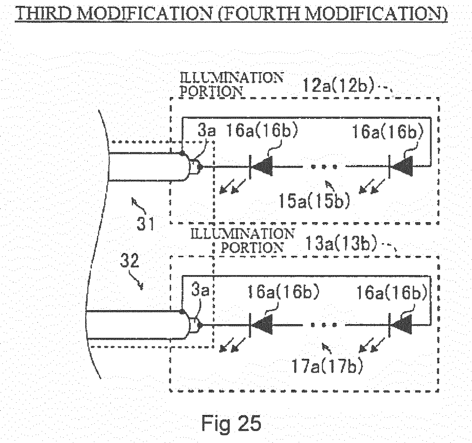

In the example shown in the above-described first to eighth embodiments, a light-emitting diode element is used as the light-emitting element according to the present invention. However, this is not to limit the present invention. A light-emitting element other than a light-emitting diode element can be used as the light-emitting element according to the present invention. For example, as in a modification shown in FIG. 25, a semiconductor laser element 16a or an organic light-emitting diode element 16b may be used as the light-emitting element.

As shown in FIG. 25, an illumination portion 12a (and an illumination portion 13a) according to a third modification includes (include) a light source portion 15a (and a light source portion 17a). The light source portion 15a (each of the light source portions 15a and 17a) includes the semiconductor laser element 16a. The semiconductor laser element 16a is capable of emitting a laser beam of relatively high directivity, compared to a light-emitting diode element. Thus, in this case, much of the beam from the semiconductor laser element 16a can be applied reliably to the test object P.

As shown in FIG. 25, an illumination portion 12b (and an illumination portion 13b) according to a fourth modification includes (include) a light source portion 15b (and a light source portion 17b). The light source portion 15b (each of the light source portions 15b and 17b) includes the organic light-emitting diode element 16b. The organic light-emitting diode element 16b can be reduced in thickness easily. Thus, in this case, the light source portion 15b (and the light source portion 17b) can be reduced easily in size. An element other than a light-emitting diode element, a semiconductor laser element, and an organic light-emitting diode element can be used as the light-emitting element.

In the example shown in the above-described first to eighth embodiments, the signal generating portion according to the present invention includes two FETs. However, this is not to limit the present invention. In the present invention, the signal generating portion can be configured to include FETs of a number other than two. For example, if the forward voltage values of light-emitting diode elements (light-emitting elements) are substantially equal (not different largely) even in the presence of two illumination portions like in the above-described first to third embodiments, one FET may be provided and the FET may be configured in such a manner that respective inner conductors of two coaxial cables are connected to the drain of the FET.

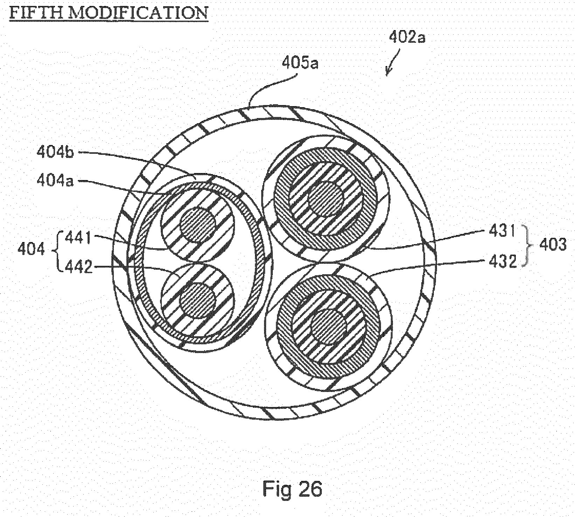

In the example shown in the above-described fourth embodiment, the cable 402 includes the shield 403a (first shield) covering the outside of the coaxial cable 403. In the example shown in the above-described fifth embodiment, the cable 502 includes both the shield 403a (first shield) covering the outside of the coaxial cable 403 and the shield 404a (first shield) covering the outside of the signal cable 404. However, these are not to limit the present invention. Like a cable 402a shown in FIG. 26 according to a fifth modification of the fourth and fifth embodiments, for example, a configuration can be such that the shield 404a (first shield) covering the outside of the signal cable 404 is provided while the shield 403a covering the outside of the coaxial cable 403 is omitted.

As shown in FIG. 26, the cable 402a according to the fifth modification includes the coaxial cable 403 and the signal cable 404. The cable 402a includes the shield 404a covering the outside of the signal cable 404. The cable 402a includes the jacket 404b covering the outer peripheral surface of the shield 404a, and a jacket 405a covering the outsides of the coaxial cable 403, the signal cable 404, the shield 404a, and the jacket 404b. In this way, the cable 402a is configured in such a manner that the coaxial cable 403 and the signal cable 404 covered by the shield 404a can be routed integrally.

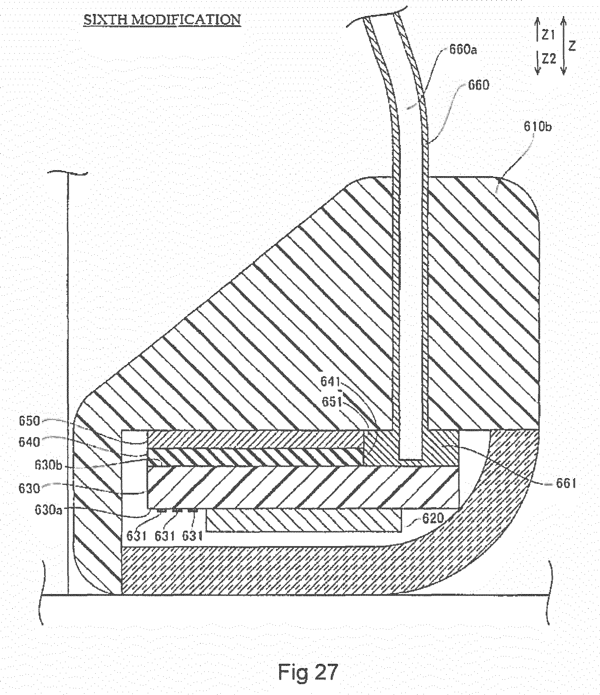

In the above-described sixth to eighth embodiments, the light source portion 620 is arranged on the first surface 630a of the substrate 630 and the wire 631 is arranged on the second surface 630b of the substrate 630. However, this is not to limit the present invention. In the present invention, as in a sixth modification shown in FIG 27, the wire 631 can be arranged on the first surface 630a of the substrate 630.

In this case, the electromagnetic wave absorption layer 950 can be provided not only on the second surface 630b of the substrate 630 but also on the first surface 630a of the substrate 630.

In the above-described sixth to eighth embodiments, the electromagnetic wave absorption layer 650 is a sheet-like member. However, this is not to limit the present invention. In the present invention, the electromagnetic wave absorption layer 650 may have a paste-like shape.

Reference Sings List

| | |
|---|---|
| 2, 2a | Apparatus body |
| 3, 31, 32, 203, 231, 232, 303, 403, 431, 432 | Coaxial cable |
| 3a, 431a, 432a | Inner conductor |
| 3c, 431c, 432c | Outer conductor |
| 4, 404, 441, 442 | Signal cable |
| 14 | Acoustic wave detecting portion (detecting portion) |
| 15, 15a, 17, 17a, 620 | Light source portion |
| 16 | Light-emitting diode element (light-emitting element) |
| 16a | Semiconductor laser element (light-emitting element) |
| 16b | Organic light-emitting diode element (light-emitting element) |
| 22, 322, 682, 922 | Light source drive portion |
| 22a, 922a | Power supply portion |
| 22b, 922b | Signal generating portion |
| 100, 200, 300, 400, 500, 600, 700, 800 | Photoacoustic imaging apparatus |
| 331 | First coaxial cable (coaxial cable) |
| 332 | Second coaxial cable (coaxial cable) |
| 333 | Third coaxial cable (coaxial cable) |
| 334 | Fourth coaxial cable (coaxial cable) |
| 403a, 404a | Shield (first shield) |
| 502a | Cable group |
| 505a | Shield (second shield) |
| 601a | Heat dissipating portion |
| 610a | First housing (housing) |
| 620a | Light-emitting element |
| 630 | Substrate |
| 630a | First surface |
| 630b | Second surface |
| 631 | Wire |
| 640 | Insulating member |
| 650 | Electromagnetic wave absorption layer |
| 651 | Substrate exposing portion |
| 670 | Detecting portion |
| 660 | Heat conducting portion |

**Claims**

1. A photoacoustic imaging apparatus (100, 200, 300, 400, 500, 600, 700, 800) comprising:

a light-emitting element (16, 16a, 16b, 620a) capable of emitting light to a test object;
a detecting portion (14, 670) that detects an acoustic wave generated by absorption of light by a detection target in the test object, the light being light emitted from the light-emitting element to the test object;
an apparatus body (2, 2a, 680) with a light source drive portion (22, 322, 682, 922) that includes a power supply portion (22a, 922a) and a signal generating portion (22b, 922b), the power supply portion supplying power to the light-emitting element, the signal generating portion generating a pulse emission signal for controlling a state of the light-emitting element of emitting light and a state of the light-emitting element of not emitting light; and
a coaxial cable (3, 31, 32, 203, 231, 232, 303, 331 to 334, 403, 431, 432) connecting the light-emitting element and the apparatus body, wherein

the coaxial cable has an outer conductor (3c, 431c, 432c) and an inner conductor (3a, 431 a, 432a), the outer conductor being connected to the power supply portion of the light source drive portion or grounded, the inner conductor being connected to the signal generating portion of the light source drive portion.

2. The photoacoustic imaging apparatus according to claim 1, wherein
the light source drive portion is configured to generate a flow of a pulsed current of 10A or more in the coaxial cable when the pulse emission signal is generated for placing the light-emitting diode element in the state of emitting light.

3. The photoacoustic imaging apparatus according to claim 1, wherein
the coaxial cable is configured to have a characteristic impedance of 30 $\Omega$ or less.

4. The photoacoustic imaging apparatus according to claim 3, wherein
the coaxial cable is configured to have a characteristic impedance of 15 $\Omega$ or more.

5. The photoacoustic imaging apparatus according to claim 1, wherein
the light-emitting element and the apparatus body are connected through a plurality of the coaxial cables.

6. The photoacoustic imaging apparatus according to claim 1, further comprising:

an imaging portion that forms an image of the acoustic wave detected by the detecting portion based on a signal of the acoustic wave; and
a signal cable (4, 404, 441, 442) connected to the imaging portion and the detecting portion and transmitting the signal of the acoustic wave, wherein
the coaxial cable and the signal cable are configured to be routed in an integrated state.

7. The photoacoustic imaging apparatus according to claim 6, further comprising a first shield (403a, 404a) covering the outside of at least one of the coaxial cable and the signal cable.

8. The photoacoustic imaging apparatus according to claim 6, wherein
the coaxial cable and the signal cable form a cable group (502a) routed in an integrated state,
the photoacoustic imaging apparatus further comprising a second shield (505a) covering the outside of the cable group.

9. The photoacoustic imaging apparatus according to claim 1, wherein
the outer conductor of the coaxial cable is connected to the power supply portion of the light source drive portion and the inner conductor of the coaxial cable is connected to the signal generating portion.

10. The photoacoustic imaging apparatus according to claim 1, wherein
the coaxial cable is configured to have a conductor resistance of 0.5 $\Omega$/m or less.

11. The photoacoustic imaging apparatus according to claim 1, further comprising:

a light source portion (620) including the light-emitting element;
a substrate (630) having a first surface (630a) and a second surface (630b) opposite the first surface, the light source portion being arranged on the first surface, a wire (631) being arranged on the first surface or the second surface; and
an electromagnetic wave absorption layer (650) provided to cover the wire from a place adjacent to the second surface of the substrate.

12. The photoacoustic imaging apparatus according to claim 11, wherein
the electromagnetic wave absorption layer includes a substrate exposing portion (651) for exposing the second surface of the substrate,
the photoacoustic imaging apparatus further comprising a heat conducting portion (660) for dissipating heat from the substrate, the heat conducting portion being arranged to contact the second surface of the substrate through the substrate exposing portion of the electromagnetic wave absorption layer.

13. The photoacoustic imaging apparatus according to claim 12, further comprising a housing (610a) housing the detecting portion, wherein
the heat conducting portion is configured to contact the second surface of the substrate at one end of the heat conducting portion and to contact the housing at an opposite end of the heat conducting portion.

**14.** The photoacoustic imaging apparatus according to claim 13, wherein
the housing includes a heat dissipating portion (601a), and
the heat conducting portion is configured to contact the heat dissipating portion at the opposite end of the heat conducting portion.

**15.** The photoacoustic imaging apparatus according to claim 11, wherein
an insulating member (640) is provided between the second surface of the substrate and the electromagnetic wave absorption layer.

**16.** The photoacoustic imaging apparatus according to claim 1, further comprising a light source portion (15, 15a, 17, 17a, 620) including the light-emitting element, wherein
the light source portion and the detecting portion are arranged adjacent to each other.

**17.** The photoacoustic imaging apparatus according to claim 1, wherein
the light-emitting element includes a plurality of light-emitting elements, and
the light-emitting elements are arranged in a linear pattern.

**18.** The photoacoustic imaging apparatus according to claim 1, wherein
the light-emitting element is formed of a light-emitting diode element (16).

**19.** The photoacoustic imaging apparatus according to claim 1, wherein
the light-emitting element is formed of a semiconductor laser element (16a).

**20.** The photoacoustic imaging apparatus according to claim 1, wherein
the light-emitting element is formed of an organic light-emitting diode element (16b).

FIRST EMBODIMENT (SECOND EMBODIMENT)

FIG 1

FIRST EMBODIMENT

Fig 2

FIRST EMBODIMENT 31 (32)

Fig 3

FIRST EMBODIMENT

Fig 4

<u>WITH USE OF COAXIAL CABLE</u>

Fig 5

WITHOUT USE OF COAXIAL CABLE (COMPARATIVE EXAMPLE)

Fig 6

SECOND EMBODIMENT

Fig 7

THIRD EMBODIMENT

300

COAXIAL CABLE 303

LIGHT SOURCE
DRIVE PORTION 322

22a

POWER
SUPPLY
PORTION

PULSE EMISSION
SIGNAL

SIGNAL
GENERATING 22b
PORTION

23

CONTROL
PORTION

ILLUMINATION
PORTION 312

16    16

315

ILLUMINATION 313
PORTION

16    16

317

Fig 8

30

FOURTH EMBODIMENT

Fig 9

FOURTH EMBODIMENT

Fig 10

FOURTH EMBODIMENT

Fig 11

FIFTH EMBODIMENT

502a · 505b · 500 · 502

505a

403b

403a

404b

404a

404 { 441
       442

431 } 403
432

Fig 12

SIXTH EMBODIMENT

Fig 13

Fig 14

SIXTH EMBODIMENT

Fig 15

SIXTH EMBODIMENT

Fig 16

SIXTH EMBODIMENT

Fig 17

Fig 18

COMPARATIVE EXAMPLE

Fig 19

SIXTH EMBODIMENT

Fig 20

SEVENTH EMBODIMENT 700

Fig 21

EIGHTH EMBODIMENT

Fig 22

FIRST MODIFICATION

Fig 23

Fig 24

THIRD MODIFICATION (FOURTH MODIFICATION)

ILLUMINATION PORTION 12a(12b)

3a 16a(16b)    16a(16b)

15a(15b)

31

32

ILLUMINATION PORTION 13a(13b)

3a 16a(16b)    16a(16b)

17a(17b)

Fig 25

FIFTH MODIFICATION

Fig 26

Fig 27

## INTERNATIONAL SEARCH REPORT

| International application No. |
|---|
| PCT/JP2015/069286 |

| A. CLASSIFICATION OF SUBJECT MATTER |
|---|
| *A61B8/13*(2006.01)i |

According to International Patent Classification (IPC) or to both national classification and IPC

| B. FIELDS SEARCHED |
|---|
| Minimum documentation searched (classification system followed by classification symbols)<br>A61B8/13 |

Documentation searched other than minimum documentation to the extent that such documents are included in the fields searched

| Jitsuyo Shinan Koho | 1922–1996 | Jitsuyo Shinan Toroku Koho | 1996–2015 |
|---|---|---|---|
| Kokai Jitsuyo Shinan Koho | 1971–2015 | Toroku Jitsuyo Shinan Koho | 1994–2015 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2010-12295 A (Toshiba Corp.),<br>21 January 2010 (21.01.2010),<br>paragraph [0069]; fig. 3 to 4<br>(Family: none) | 1-20 |
| A | JP 2013-208230 A (Fujifilm Corp.),<br>10 October 2013 (10.10.2013),<br>paragraphs [0020], [0031]; fig. 3<br>& US 2015/0018687 A1 & CN 104203107 A | 1-20 |
| A | JP 2014-532520 A (Seno Medical Instruments, Inc.),<br>08 December 2014 (08.12.2014),<br>paragraphs [0012] to [0013]; fig. 1 to 2<br>& US 2013/0109950 A1 & WO 2013/067304 A1 | 1-20 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 September 2015 (04.09.15) | 15 September 2015 (15.09.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

**INTERNATIONAL SEARCH REPORT**

International application No.

PCT/JP2015/069286

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2014-39801 A (Fujifilm Corp.),<br>06 March 2014 (06.03.2014),<br>paragraphs [0026] to [0097]; fig. 1 to 15<br>& WO 2014/017044 A | 1-20 |

Form PCT/ISA/210 (continuation of second sheet) (July 2009)

**EP 3 181 057 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013188330 A
- JP 2008044148 A